(19) Europäisches Patentamt
European Patent Office
Office européen des brevets

(11) Publication number: **0 218 300 A2**

(12) # EUROPEAN PATENT APPLICATION

(21) Application number: **86201710.0**

(22) Date of filing: **12.05.83**

(51) Int. Cl.⁴: **C 07 D 215/54**, C 07 D 401/12, C 07 D 417/12, C 07 D 401/14, C 07 J 43/00, A 61 K 47/00

(30) Priority: **18.05.82 US 379316**
**27.01.83 US 461543**
**15.03.83 US 475493**

(43) Date of publication of application: **15.04.87**
**Bulletin 87/16**

(84) Designated Contracting States: **AT BE CH DE FR GB LI LU NL SE**

(60) Publication number of the earlier application in accordance with Art. 76 EPC: **0110955**

(71) Applicant: **UNIVERSITY OF FLORIDA, 207 Tigert Hall, Gainesville Florida 32611 (US)**

(72) Inventor: **Bodor, Nicholas S., 7211 South West 97th Lane, Gainesville Florida 32608 (US)**

(74) Representative: **Pendlebury, Anthony et al, Page, White & Farrer 5 Plough Place New Fetter Lane, London EC4A 1HY (GB)**

(54) **Brain-specific drug delivery.**

(57) The invention provides compounds adapted for the site-specific/sustained delivery of a centrally acting drug species to the brain having the formula [D–DHC] and the non-toxic pharmaceutically acceptable salts thereof, wherein [D] is a centrally acting drug species containing at least one reactive –OH, –COOH, –SH, –NH– or $-NH_2$ functional group and [DHC] is the reduced, biooxidizable, blood-brain barrier penetrating lipoidal form of a dihydropyridine ⇌ pyridinium salt redox carrier comprising a dihydroquinoline or dihydroisoquinoline ring system, a carbon atom of the nitrogen-containing ring portion of said ring system being connected via a bridging group to a reactive –OH, –COOH, –SH, –NH– or $-NH_2$ functional group in the centrally acting drug species. The corresponding quaternary derivatives are also described.

EP 0 218 300 A2

Indeed, the barriers separating plasma from the brain and cerebrospinal fluid (CSF) are complex systems involving passive and active transport and subserve a number of important functions. The boundary between plasma and the central nervous system (CNS) is much less permeable than that between plasma and other tissue cells to a variety of water soluble substances, such as organic electrolytes, organic acids and bases, as well as to large molecules such as proteins. Such a barrier also provides a path for clearance from the brain of the breakdown products of cellular metabolism. The CNS and its fluids can be considered basically a three-compartment system: the blood or the plasma, CSF and brain tissue. There is a diffusion-controlled exchange between CSF and the extracellular fluid (CF) of the brain. It has also been suggested that the permeabilities of blood-CSF and blood-brain barriers are practically identical with respect to drugs and other foreign substances. Mayer et al, J. Pharmacol. and Exp. Therap., 125, 185 (1959).

The BBB is, moreover, basically the result of the fact that the endothelial cells in the brain capillaries are joined by continuous, tight intercellular junctions, such that material has to pass through the cells rather than between them in order to move from blood to brain. It is interesting that there are areas within the brain, such as the subfornical body and the postremia in which the capillary cells are not closely linked so that they lack the characteristics of the BBB. They provide the entry of small amounts of compounds which would not ordinarily enter the barriers. Hoffmann and Olszewzki, Neurology (Minneap.), 11, 1081 (1961).

# BRAIN-SPECIFIC DRUG DELIVERY

## Field of the Invention:

The present invention relates to a dihydropyridine/pyridinium salt type of redox system for the site-specific or sustained delivery (or both) of a wide variety of drug species to the brain. More especially, this invention relates to the discovery that a biologically active compound coupled to a lipoidal carrier moiety comprising a dihydropyridine nucleus readily and easily penetrates the blood-brain barrier ("BBB") and attains increased levels of concentration in the brain; oxidation of the dihydropyridine carrier moiety in vivo to the ionic pyridinium salts prevents its elimination from the brain, while elimination from the general circulation is accelerated, resulting in significant and prolongedly sustained brain-specific drug activity, whether ascribable to the cleavage of the $[D-QC]^+$ entity and sustained release of the drug in the brain and/or to $[D-QC]^+$ itself.

## Background Art:

The delivery of drug species to the brain is ofttimes seriously limited by transport and metabolism factors and, more specifically, by the functional barrier of the endothelial brain capillary wall deemed the blood-brain barrier, BBB. Site-specific delivery and sustained delivery of drugs to the brain are even more difficult, and to date no useful simple or generic techniques to achieve such phenomena are known to the art.

Foreign compounds which enter organs other than the central nervous system with ease, may penetrate the CNS slowly or hardly at all. A number of theories concerning the nature of the barrier have been proposed. The widely accepted concept describes the boundary as a fat-like layer interspersed with small pores, although the BBB is not a simple, anatomically well-defined unitary physical entity. Shuttleworth, Prog. Exp. Tumor Res., 17, 279 (1972). Penetration of such a barrier may occur by several processes: lipid soluble substances may passively penetrate into the cells, while small molecules such as water and urea may pass through the pores. In addition to these simple physical processes, carrier-mediated and active transport processes govern the movement of many molecules through the BBB. Thus, it is generally accepted that lipid solubility, degree of ionic dissociation or protonation and the ability of temporary combination with membrane constituents affect delivery through the BBB. It has been shown, for example, that in the class of barbiturates, a quantitative correlation could be established between their ease to pass into the brain (as reflected by the different times of onset of anesthetic action) and their lipid/water partition coefficient. Mark et al, J. Pharmacol. and Exp. Therap., 123, 79 (1957). The role of lipid solubility in drug penetration through the BBB is also exemplified by the better absorption of the sparingly water-soluble thiamine propyl disulfide (TPD) as compared to the water-soluble thiamine hydrochloride (THCl). Thomson et al, Ann. Int. Med., 74, 529 (1971). Some materials such

as glucose and amino acids are transported by active mechanism, characterized by saturation, bidirectional molecular specificity, bidirectional competitive inhibition and bidirectional countertransport. Fishman, Am J. Physiol., 206, 836 (1964).

Changes in permeability of the BBB can be caused by several pathological and toxicological processes. Pardridge, Connor and Crawford, CRC Crit. Rev. Toxicol., 179 (1975). A general increase in the barrier permeability, such as a nonspecific breakdown of the barrier has, however, severe consequences, including cerebral edema.

It too is well documented that the BBB is relatively impermeable to the ionized forms of drugs and other molecules. Drugs which are weak organic electrolytes appear to pass from blood to CSF to reach a steady state ratio characteristic of each molecule according to its $pK_a$ and the existence of a normal pH gradient between blood and CSF. It is clear that it is the most difficult for quaternary pyridinium or ammonium salts to penetrate the BBB.

And removal of substances from the brain and CSF is obviously a significant factor in regulating drug concentrations in the CNS. There are several efflux processes: bulk flow via the arachnoid villi, diffusion of lipid soluble substances into brain and blood, active transport and metabolism by adjacent meninges. Once a drug or metabolite enters the CSF from blood or brain by simple diffusion, it may rapidly be removed, either by nonselective bulk flow or by active transport mechanism associated with the choroid plexus or other nondefined structures in the CSF compartment. It is generally accepted that highly lipid-soluble drugs

leave the CSF more rapidly than poorly lipid-soluble ones, but the barrier to passage of compounds from CSF has only superficial similarity to the blood-CSF barrier.

Drug elimination processes from the brain are significantly directly related to drug accumulation in the brain. It is generally assumed that efflux in the opposite direction involves almost the same processes as for entry, except that the role of the bulk flow and the metabolic processes in the brain are not to be overlooked.

The two elimination processes studied in the earlier literature and which can be said to have a certain bearing on the present invention involve elimination from the brain of ionic species. Thus, it is found that non-metabolized ionic species, such as the acetate ion, have a three times slower elimination rate from the CSF than from the blood. Freundt, Arz., Forsch., 23, 949 (1973). An even more dramatic change in the elimination rate was found in the case of a quaternary piperidinium salt. The quaternary salt, formed *in situ* after delivery of a haloalkylamine, which undergoes cyclization to the quaternary salt, in the brain, as well, was found to have an at least ten times slower elimination rate from the brain than from the rest of the body. It was concluded by the authors (Ross and Froden, Eur. J. Pharmacol., 13, 46 [1970] that the outflow rate of the quaternary salt corresponded to the inflow rate. Similar results were obtained for the erythrocytes: the efflux of the quaternary salt was very slow. Ross, J. Pharm. Pharmacol., 27, 322 (1975).

And while it too has been suggested to deliver a drug species, specifically N-methylpyridinium-2-carbaldoxime chloride (2-PAM), into the brain, the active nucleus of which in and of itself constituting a quaternary pyridinium salt, by way of the dihydropyridine latentiated prodrug form thereof, such approach is conspicuously delimited to relatively small molecule quaternary pyridinium ring-containing drug species and does not provide the overall ideal result of brain-specific, sustained release of the desired drug, with concomitant rapid elimination from the general circulation, enhanced drug efficacy and decreased toxicity. Hence, no "trapping" in the brain of the 2-PAM formed _in situ_ results, and obviously no brain-specific, sustained delivery occurs as any consequence thereof: the 2-PAM is eliminated as fast from the brain as it is from the general circulation and other organs. Compare U.S. Patents Nos. 3,929,813 and 3,962,447; Bodor et al, _J. Pharm. Sci._, _67_, No. 5, 685 (1978). It has also been speculated to deliver, e.g., an antitumor agent into the brain by utilizing a dihydropyridine/pyridinium redox carrier moiety therefor, but this particular hypothesis necessarily entails derivatizing the dihydropyridine/pyridinium carrier with a substituent $R_1$ itself critically designed to control the release rate of the active drug species from the quaternary drivative thereof, as well as being critically functionally coordinated with the particular chemical and therapeutic activity/nature of the antitumor drug species itself; Bodor et al, _J. Pharm. Sci._, supra.

Accordingly, acutely serious need exists in this art for a truly effective generic but nonetheless flexible method for the site-specific, or sustained delivery, or both, of drug species to the brain, while at the same time avoiding the aforesaid noted and notable disadvantages and drawbacks associated with penetration of the blood-brain barrier, with dihydropyridine latentiated prodrug forms of drug species themselves comprising a pyridinium salt active nucleus, and with the necessity for introducing critically coordinated and designed, release rate-controlling substituents onto any particular drug carrier moiety.

Recently, Bodor et al, Science, Vol. 214, December 18, 1981, pp. 1370-1372, have reported on site-specific sustained release of drugs to the brain. The Science publication outlines a scheme for specific and sustained delivery of drug species to the brain, as depicted in the following Scheme 1:

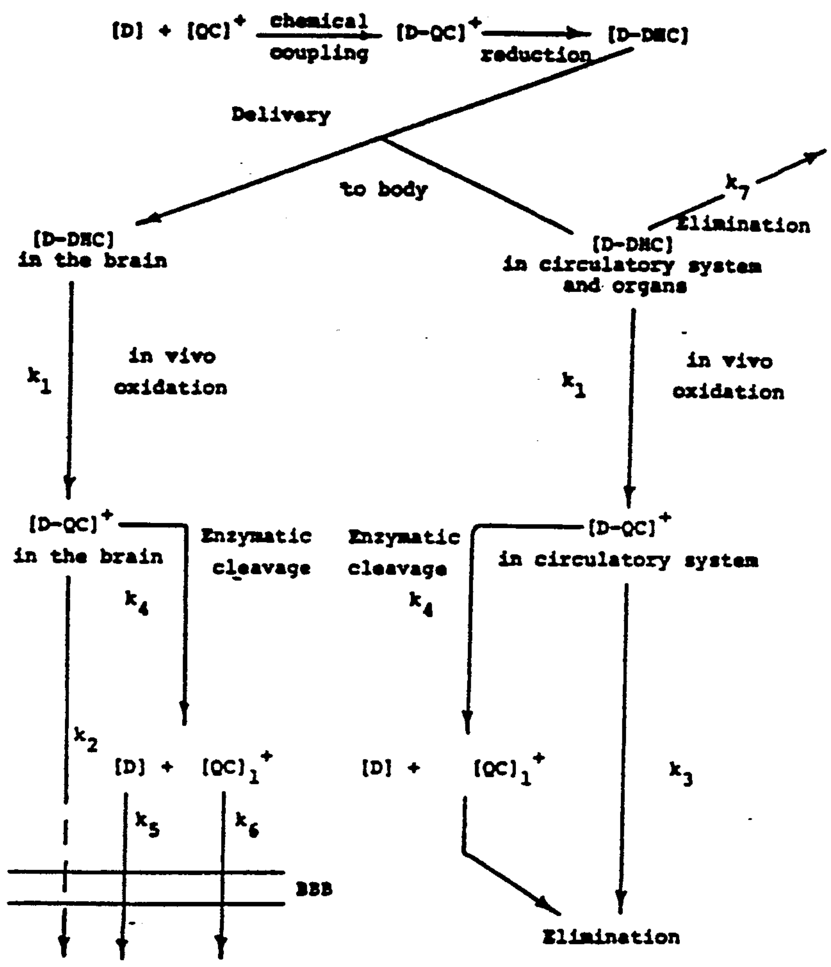


Scheme 1: BBB, blood-brain barrier.

Bodor et al have reported, in Science, their work with phenylethylamine as the drug model, which was coupled to nicotinic acid, then quaternized to give compounds of the formula

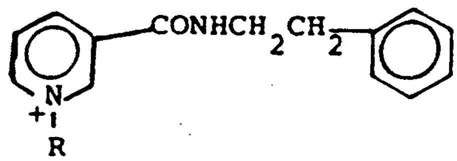 (R=$CH_3$ or $CH_2$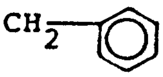 )

which were subsequently reduced by sodium dithionite to the corresponding compounds of the formula

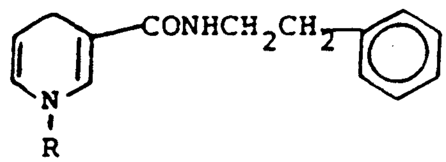 (R=$CH_3$ or $CH_2$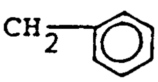 ).

Testing of the N-methyl derivative in vivo supported the criteria set forth in Scheme 1. Bodor et al speculated that various types of drugs might possibly be delivered using the depicted or analogous carrier systems and indicated that use of N-methylnicotinic acid esters and amides and their pyridine ring-substituted derivatives was being studied for delivery of amino- or hydroxyl-containing drugs, including small peptides, to the brain. No other possible specific carriers were disclosed.

Other reports of Bodor et al's work have appeared in The Friday Evening Post, August 14, 1981, Health Center Communications, University of Florida, Gainesville, Florida; Chemical & Engineering News, December 21, 1981, pp. 24-25; and Science News, January 2, 1982, Vol. 121, No. 1, page 7. These publications do not suggest any carrier systems other

According to the scheme in Science, a drug [D] is coupled to a quaternary carrier $[QC]^+$ and the $[D-QC]^+$ which results is then reduced chemically to the lipoidal dihydro form [D-DHC]. After administration of [D-DHC] *in vivo*, it is rapidly distributed throughout the body, including the brain. The dihydro form [D-DHC] is then *in situ* oxidized (rate constant, $k_1$) (by the NAD $\rightleftharpoons$ NADH system) to the ideally inactive original $[D-QC]^+$ quaternary salt which, because of its ionic, hydrophilic character, should be rapidly eliminated from the general circulation of the body, while the blood-brain barrier should prevent its elimination from the brain ($k_3 >> k_2$; $k_3 >> k_7$). Enzymatic cleavage of the $[D-QC]^+$ that is "locked" in the brain effects a sustained delivery of the drug species [D], followed by its normal elimination ($k_5$), metabolism. A properly selected carrier $[QC]^+$ will also be rapidly eliminated from the brain ($k_6 >> k_2$). Because of the facile elimination of $[D-QC]^+$ from the general circulation, only minor amounts of drug are released in the body ($k_3 >> k_4$); [D] will be released primarily in the brain ($k_4 > k_2$). The overall result ideally will be a brain-specific sustained release of the target drug species.

than the specific N-methyl and N-benzyl nicotinic acid-type carriers disclosed in the Science publication. Other classes of drugs as well as a few specific drugs are mentioned as possible candidates for derivativization; for example, steroid hormones, cancer drugs and memory enhancers are indicated as targets for possible future work, as are enkephalins, and specifically, dopamine and testosterone. The publications do not suggest how to link such drugs to the carrier, except possibly when the drugs are simple structures containing a single $NH_2$ or, perhaps, simple structures containing a single OH, of the primary or secondary type, as is the case with phenylethylamine or testosterone. There is, for example, no suggestion of how one of ordinary skill in the art would form a drug-carrier combination when the drug has a more complicated chemical structure than phenylethylamine, e.g., dopamine or an enkephalin. Thus, except in a very limited area, where only a combination of certain types of drug structures with certain utility classes and certain carrier structures is taught or suggested, the publications simply do not make the broad concept of the invention as represented by Scheme 1 available to the public. Likewise, the many embodiments of the invention as described hereinafter are unsuggested by the art.

It is also known to this art that Parkinsonism, a striatal dopamine deficiency syndrome [H. Ehringer and O. Hornykiewicz, Klin. Wsch., 38, 1236 (1960)],

cannot be treated directly with dopamine, for dopamine and related catecholamines also do not cross the blood-brain barrier [B. E. Roos and G. Steg, Life Sci., 3, 351 (1964)]. L-Dopa, considered as a prodrug for dopamine, was first discovered to be useful in the treatment of Parkinsonism more than twenty years ago [A. Barbeau, Excepta Medica, Int. Congr. Ser., 38, 152 (1961); W. Birkmayer and O. Hornykiewicz, Wien. Klin. Wochnenschr., 73, 787 (1961)]. Indeed, L-Dopa is considered to be the best available treatment for Parkinsonism, but, unfortunately, at the expense of a wide variety of undesirable side effects [A.Barbeau, TIPS, 2, (11), 297 (1981)]. The peripheral side effects of L-Dopa, which range from nausea and vomiting to cardiac arrythmias and hypotension, appear to be due to one or more of the metabolic products thereof, rather than L-Dopa per se. L-Aromatic amino acid decarboxylase enzyme is responsible for the major metabolism of L-Dopa, whether prior, during or after absorption. Concurrent administration of L-Dopa with an inhibitor of aromatic amino acid decarboxylase, which should not be able to penetrate the BBB, reduces the decarboxylation of L-Dopa in peripheral tissues. Such reduction allows higher proportions of L-Dopa to reach the CNS and at the same time diminishes the peripheral side effects considerably, particularly vomiting and cardiac arrythmias, but a number of serious side effects still persist [A.Barbeau, TIPS, supra; A.Barbeau and M.Roy, Neurology, 26, 399 [(1976)].

Attempts have also been made to alleviate the well-known dissolution, absorption and metabolism problems of L-Dopa [H. Ninterberger, Biochem. Med., 5, 412 (1971); H. Shindo, T. Komai, K. Tanaka, E. Nakajima and N. Miyakoshi, Chem. Pharm. Bull., 21, 826 (1973); C. O. Rutledge and M. M. Hoehn, Nature (London), 244, 447 (1973); R. L. Bronaugh, R. J. McMurty, M. M. Hoehn and C. O. Rutledge, Biochem. Pharmacol., 24, 1317 (1975)], employing prodrug approaches [N. Bodor, K. B. Sloan, T. Higuchi and K. Sasahara, J. Med. Chem., 20, 1435 (1977); A. M. Felix, D. P. Winter, S. S. Wang, I. D. Kulesha, W. R. Pool, D. L. Hane and H. Sheppard, J. Med. Chem., 17, 422 (1974)].

Additionally, dopamine agonists, which are used in the treatment of hyperprolactinemia associated with pituitary adenomas or amenorrhea [R. F. Spark and G. Dickenstein, Ann. Int. Med., 90, 949 (1979)], also induce unwanted side effects.

Thus, especially acutely serious need exists in this art to delivery a dopaminergic agent directly and specifically to the brain, in a sustained manner, and there elicit the desired dopaminergic response, e.g., for the treatment of Parkinsonism or hyperprolactinemia.

~~The existence of a blood-testis barrier (BTB) by which some substances are prevented from being carried into the seminiferous tubules long has been suspected [P.P.H. Bruyn, R.C. Robertson and R.S. Farr, Anat. Rec., 108, 279 (1950); R.J. Goldacre and B. Sylven, Nature (London), 184, 63 (1959); R.J. Goldacre and B. Sylven, J. Cancer, 16, 306 (1962); T.S. Ro and H. Busch, Biochem. Biophys Acta (Amst), 108, 317 (1965)].~~

to achieve such results are known to the art. It is thus apparent that a serious need exists in this art for a method for the site-specific and/or sustained delivery of drug species to the testes to elicit the desired therapeutic, e.g., hormonal or tumor-inhibiting, response.

Summary and Objects of the Invention:

Accordingly, a major object of the present invention is the provision of a generic method for the specific and/or target enhanced delivery to the brain of a wide variety of drug species and to achieve brain-specific drug delivery by effecting the bidirectional transport of the drug species into and out of the brain employing dihydropyridine $\rightleftharpoons$ pyridinium salt carrier type redox systems.

Another object of the invention is to provide for brain specific drug delivery utilizing a dihydropyridine $\rightleftharpoons$ pyridinium salt carrier type redox system, which drug/carrier system is characterized by enhanced drug efficacy and decreased toxicity. Indeed, consistent herewith systemic toxicity is significantly reduced by accelerating the elimination of the drug/quaternary carrier system, and even central toxicity is reduced by providing a low level, sustained release of the active drug species in the brain.

Yet another object of this invention is the provision of a chemical delivery system for the site-specific and sustained release of drug species to the brain, and one in which a special pro-prodrug reduced

form of an active drug species is actually delivered to the body of a patient, not a prodrug as such and not a drug/carrier entity necessarily comprised of critically tailored release rate-controlling substituent(s).

Yet another object of this invention is to provide enhanced delivery to the brain of a wide variety of centrally acting agents which are not themselves able to penetrate the blood-brain barrier to any considerable extent.

In accord with the foregoing, the present invention provides compounds adapted for the site-specific/sustained delivery of a centrally acting drug species to the brain, said compounds being:

compounds of the formula

$$[D\text{-}DHC] \qquad (I)$$

and the non-toxic pharmaceutically acceptable salts thereof, wherein [D] is a centrally acting drug species containing at least one reactive -OH, -COOH, -SH, -NH- or $-NH_2$ functional group (including compounds such as amides, imides, hydrazines and guanidines, as well as compounds having a primary or secondary amino group), and [DHC] is the reduced, biooxidizable, blood-brain barrier penetrating lipoidal form of a dihydropyridine $\rightleftharpoons$ pyridinium salt redox carrier

comprising a dihydroquinoline or dihydroisoquinoline ring system, a carbon atom of the nitrogen-containing ring portion of said ring system being connected via a bridging group to a reactive -OH, -COOH, -SH, -NH- or $-NH_2$ functional group in the centrally acting drug species.

In another aspect, the present invention provides compounds having the formula

$$[D\text{-}QC]^+ \qquad (II)$$

wherein [D] is a centrally acting drug species containing at least one reactive -OH, -COOH, -SH, -NH- or $-NH_2$ functional group (including compounds such as amides, imides, hydrazines and guanidines, as well as compounds having a primary or secondary amino group), and $[QC]^+$ is the hydrophilic, ionic pyridinium salt form of a dihydropyridine $\rightleftharpoons$ pyridinium salt redox carrier

comprising a dihydroquinolinium or dihydroisoquinolinium ring system, a carbon atom of the nitrogen-containing ring portion of said ring system being connected via a bridging group to a reactive -OH, -COOH, -SH, -NH- or $-NH_2$ functional group in the centrally acting drug species.

In yet another aspect, the present invention provides, as an effective dopaminergic chemical delivery system, compounds having the formula

[D-DHC] (I)

and non-toxic pharmaceutically acceptable salts thereof, wherein [D] is a dopamine having the structural formula

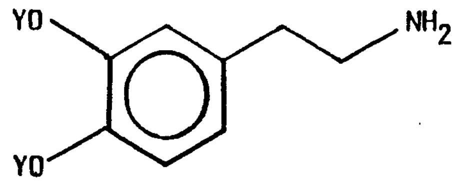

in which each Y is independently a hydrolytically or metabolically cleavable hydroxyl protective group, and [DHC] is defined as above.

In still another aspect, the present invention provides compounds having the formula

$$[D\text{-}QC]^+ \qquad (II)$$

wherein [D] is a dopamine having the structural formula

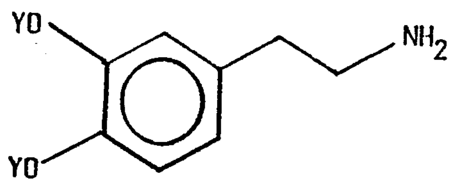

in which each Y is a hydrolytically or metabolically cleavable hydroxyl protective group, and $[QC]^+$ is defined as above.

Briefly, in a chemical delivery system for dopamine according to this invention, the amino function of dopamine is appropriately linked to the dihydropyridine-type carrier system, while the catechol function is advantageously protected, for example, as a corresponding ester function, e.g., the dipivalyl ester. The brain-specific delivery of dopamine, or the otherwise eliciting of a dopaminergic response, requires a succession of processes, including oxidation of the dihydropyridine ring to the corresponding pyridinium salt which provides the basis for "locking-in" the brain the molecule, hydrolysis of the, e.g., pivalyl esters likely via the 3- and/or 4-monopivalyl esters and, finally, the release of dopamine,

**Detailed Description of the Invention:**

More particularly in accord with the present invention, the following definitions are applicable:

The term "lipoidal" as used herein is intended to designate a carrier moiety which is lipid-soluble or lipophilic.

The expression "hydroxyl protective group" is intended to designate a group which prevents premature metabolism of an OH group or groups prior to the compound's reaching the desired site in the body. Typical hydroxyl protective groups contemplated by the present invention (e.g., for Y in the case of the dopamine derivatives) are acyl groups and carbonates.

When the hydroxyl protective group is acyl (i.e., when it is an organic radical derived from a carboxylic acid by removal of the hydroxyl group), it preferably represents an acyl radical selected from the group consisting of alkanoyl having 2 to 8 carbon atoms; alkenoyl having one or two double bonds and 3 to 8 carbon atoms;

$$\text{cycloalkyl-}C_nH_{2n}\text{-}\overset{\overset{\displaystyle O}{\|}}{C}\text{-}$$

wherein the cycloalkyl portion contains 3 to 7 ring atoms and n is zero, one, two or three; phenoxyacetyl; pyridinecarbonyl; and

$$\text{phenyl-}C_nH_{2n}\text{-}\overset{\overset{\displaystyle O}{\|}}{C}\text{-}$$

wherein n is zero, one, two or three and phenyl is unsubstituted or is substituted by 1 to 3 alkyl

4, which can be either a hydrolysis or a reductive process [a possible reductive release of dopamine was very recently suggested by a model for a presynaptic terminal, L. L. Miller, A. N. K. Lau and E. K. Miller, J. Am. Chem. Soc., 104, 5242 (1982)].

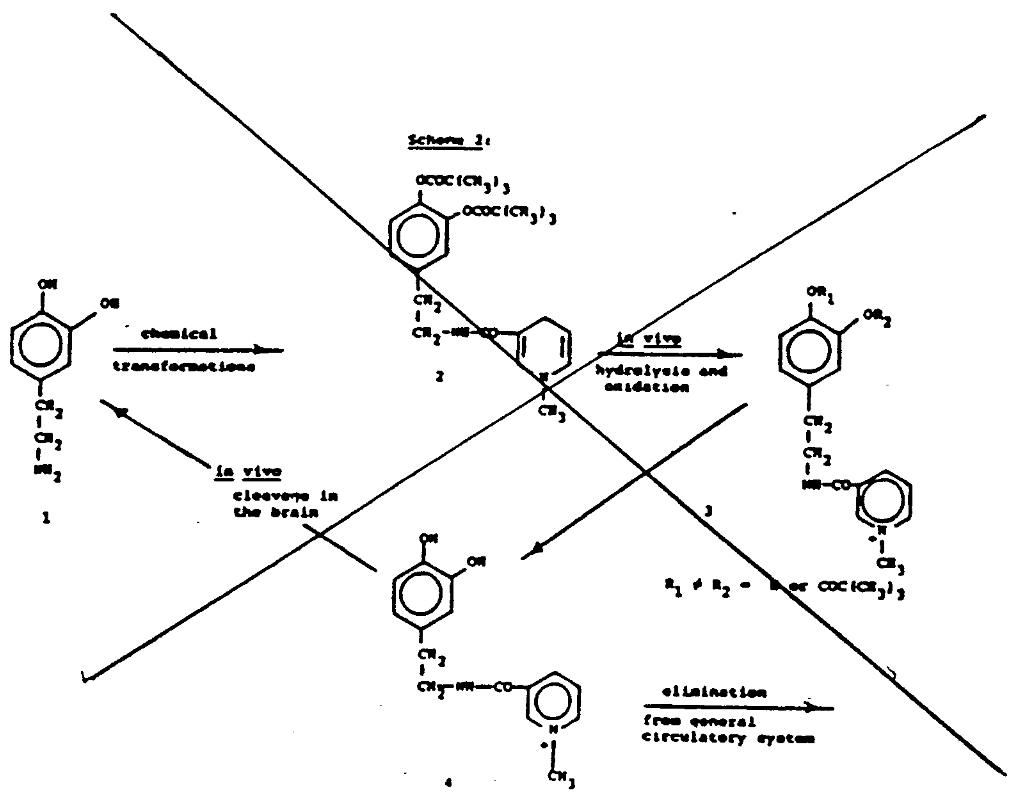

each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms.

When the acyl group is alkanoyl, there are included both unbranched and branched alkanoyl, for example, acetyl, propionyl, butyryl, isobutyryl, valeryl, isovaleryl, 2-methylbutanoyl, pivalyl (pivaloyl), 3-methylpentanoyl, 3,3-dimethylbutanoyl, 2,2-dimethylpentanoyl and the like. Pivalyl and isobutyryl are especially preferred.

When the acyl group is alkenoyl, there are included, for example, crotonyl, 2,5-hexadienoyl and 3,6-octadienoyl.

When the acyl group is

$$\text{cycloalkyl}-C_nH_{2n}-\overset{O}{\overset{\|}{C}}-$$

there are included cycloalkanecarbonyl and cycloalkanealkanoyl groups wherein the cycloalkane portion can optionally bear 1 or 2 alkyl groups as substituents, e.g. cyclopropanecarbonyl, 1-methylcyclopropanecarbonyl, cyclopropaneacetyl, α-methylcyclopropaneacetyl, 1-methylcyclopropaneacetyl, cyclopropanepropionyl, α-methylcyclopropanepropionyl, 2-isobutylcyclopropanepropionyl, cyclobutanecarbonyl, 3,3-dimethylcyclobutanecarbonyl, cyclobutaneacetyl, 2,2-dimethyl-3-ethylcyclobutaneacetyl, cyclopentanecarbonyl, cyclohexaneacetyl, cycloheptanecarbonyl and cycloheptanepropionyl.

When the acyl group is pyridinecarbonyl, there are included picolinoyl (2-pyridinecarbonyl), nicotinoyl (3-pyridinecarbonyl) and isonicotinoyl (4-pyridinecarbonyl).

When the acyl group is

$$\text{phenyl-}C_nH_{2n}\text{-}\overset{O}{\overset{\|}{C}}\text{-}$$

there are included, for example, benzoyl, phenylacetyl, α-phenylpropionyl, β-phenylpropionyl, p-toluyl, m-toluyl, o-toluyl, o-ethylbenzoyl, p-tert-butylbenzoyl, 3,4-dimethylbenzoyl, 2-methyl-4-ethylbenzoyl, 2,4,6-trimethylbenzoyl, m-methylphenylacetyl, p-isobutylphenylacetyl, β-(p-ethylphenyl)-propionyl, p-anisoyl, m-anisoyl, o-anisoyl, m-isopropoxybenzoyl, p-methoxyphenylacetyl, m-isobutoxyphenylacetyl, m-diethylaminobenzoyl, 3-methoxy-4-ethoxybenzoyl, 3,4,5-trimethoxybenzoyl, p-dibutylaminobenzoyl, p-n-butoxybenzoyl, 2,4,6-triethoxybenzoyl, 3,4-diethoxyphenylacetyl, β-(3,4,5-trimethoxyphenyl)propionyl, o-iodobenzoyl, m-bromobenzoyl, p-chlorobenzoyl, p-fluorobenzoyl, 2-bromo-4-chlorobenzoyl, 2,4,6-trichlorobenzoyl, p-chlorophenylacetyl, α-(m-bromophenyl)propionyl, p-trifluoromethylbenzoyl, 2,4-di(trifluoromethyl)benzoyl, m-trifluoromethylphenylacetyl, β-(p-trifluoromethylphenyl)propionyl, 2-methyl-4-methoxybenzoyl, 3-chloro-4-ethoxybenzoyl, β-(3-methyl-4-chlorophenyl)propionyl, p-dimethylaminobenzoyl, p-(N-methyl-N-ethylamino)benzoyl, o-acetamidobenzoyl, m-propionamidobenzoyl, 3-chloro-4-acetamidophenylacetyl and p-acetamidophenylpropionyl.

When the hydroxyl protective group is a carbonate grouping, it has the structural formula

$$Y'-O-\overset{\overset{\displaystyle O}{\|}}{C}-$$

i.e., it is an organic radical which can be considered to be derived from a carbonic acid by removal of the hydroxyl group from the COOH portion. Y' preferably represents alkyl having 1 to 7 carbon atoms; alkenyl having one or two double bonds and 2 to 7 carbon atoms;

$$\text{cycloalkyl-}C_nH_{2n}-$$

wherein the cycloalkyl portion contains 3 to 7 ring atoms and n is zero, one, two or three; phenoxy; 2-, 3- or 4-pyridyl; or

$$\text{phenyl-}C_nH_{2n}-$$

wherein n is zero, one, two or three and phenyl is unsubstituted or is substituted by 1 to 3 alkyl each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms. Most preferably, Y' is $C_1$-$C_7$ alkyl, particularly ethyl or isopropyl.

Similarly, the expression "carboxyl protective group" is intended to designate a group which prevents premature metabolism of a COOH group or groups prior to the compound's reaching the desired site in the body. Typical carboxyl protecting groups are the

groups encompassed by Y' above, especially $C_1$-$C_7$ alkyl, particularly ethyl or isopropyl.

By "centrally acting" drug species, active agent or compound as utilized herein, there is of course intended any drug species or the like, the principal pharmacological activity of which is CNS and a result of direct action in the brain.

Exemplary such centrally acting drug species are the CNS-amines and other nervous system agents, whether sympathetic or parasympathetic, e.g., phenylethylamine, dopamine, tyramine, L-DOPA, muscle relaxants, tranquilizers and antidepressants, e.g., benzodiazepine tranquilizers such as diazepam and oxazepam, phenothiazine tranquilizers such as carphenazine, fluphenazine and the like, mild and strong analgesics and narcotics, sedatives and hypnotics, narcotic antagonists, vascular agents, stimulants, anesthetics, small peptides, such as the di-, tri, tetra- and pentapeptides, and other small 6-20 aminoacid unit containing peptides, e.g., the enkephalins (for example, Tyr-Gly-Gly-Phe-Leu), which, besides being analgesics, initiate epileptic activity in the brain at doses that are about tenfold lower than for effecting analgesic activity, larger peptides, such as pituitary hormones and related agents, antiepileptic and anticonvulsant drugs generally, including hydantoins such as phenytoin and ethotoin, barbiturates such as phenobarbital, hormones, such as the steroid hormones, e.g., estradiol, testosterone, 17 α-ethynyl testosterone, and the like (recent studies on

histological mapping of hormone-sensitive and specific steroid binding cells in the brain have underscored the importance of the steroid action in the brain on sexual behavior), amphetamine-like drugs, anticancer and anti-Parkinsonism agents, antihypertensives, agents to enhance learning capacity and the memory processes, including treatment of dementias, such as Alzheimer's disease, antibacterials, centrally active hypotensive agents, diagnostic agents, such as radiopharmaceuticals, monoamine oxidase (MAO) inhibitor drugs, CNS or brain important/essential amino acids, such as tryptophan, and any like centrally acting compounds.

Other illustrative ultimate species of centrally active drug entities are: amphetamine, dextroamphetamine, levamphetamine, methamphetamine, phenmetrazine and phentermine, which are stimulants and appetite suppressants; codeine, oxycodone, pentazocine, anileridine, hydromorphone, morphine and oxymorphone, which are narcotic analgesics; desipramine, nortriptyline, opipramol and protriptyline, which are stimulants used, e.g., in endogenous depressions; clonidine and methyldopa, which are sympatholytic agents used, e.g., in hypertension; biperiden, cycrimine and procyclidine, which are centrally acting anticholinergics; tranylcypromine, a sympathomimetic stimulant and MAO inhibitor; acetophenazine, carphenazine, fluphenazine, perphenazine and piperacetazine, which are phenothiazine-type tranquilizers; chlordiazepoxide, clorazepate, nitrazepam and temazepam, which are benzodiazepine-type tranquilizers; haloperidol and clopenthixol, which are tranquilizers; norepinephrine, a sympathetic stimulant/ adrenergic agent; nalorphine and naloxone, narcotic

antagonists; hydralazine, a hypotensive; ethotoin, phenobarbital and aminoglutethimide, anticonvulsants; ethamivan, a medullary stimulant; bemegride, a barbiturate antagonist; amiphenazole, a stimulant; iopydol, iodopyracet, iodouppurate, iodamide and iopanoic acid, which are radiodiagnostics; ephedrine, pseudoephedrine, oxymetazoline and phenylephrine, which are sympathomimetic amines and decongestants; estradiol, estrone and estriol, the natural estrogens; amoxicillin, oxacillin, carbenicillin and ampicillin, pencillin-type antibiotics; amobarbital, a sedative; trihexyphenidyl, a centrally acting anticholinergic; hydroxyzine, a tranquilizer; chlortetracycline, tetracycline and methacycline, which are tetracycline-type antibiotics; clindamycin, lincomycin, nalidixic acid, oxolinic acid and phenazopyridine, antibacterials/antibiotics; flurazepam, bromazepam and lorazepam, tranquilizers; phenytoin, an anticonvulsant; glutethimide, a mild hypnotic/sedative; bethanidine and guanethidine, hypotensives/sympatholytics; methyprylon; propranolol, a β-blocker antihypertensive; dicloxacillin, an antibacterial; butalbital, a barbiturate sedative; GABA, γ-vinyl GABA, γ-acetylenic GABA, neurotransmitters for possible use in epilepsy; valproic acid and its metabolites such as 5-hydroxy-2-n-propylpentanoic acid, 4-hydroxy-2-n-propylpentanoic acid, 3-hydroxy-2-n-propylpentanoic acid, for use as anticonvulsants; apomorphine, a narcotic depressant; methotrexate, podophyllotoxin derivatives (etoposide, teniposide), doxorubicin, daunomycin and cyclophosphamide, anticancer/antitumor agents; methylphenidate, a stimulant; thiopental, an anesthetic; naloxone, a narcotic antagonist; ethinyl estradiol and mestranol, estrogens;

norgestrel and norethindrone, progestins; cephalothin, cephalexin and cefoxitin, cephalosporin antibiotics; atenolol and metoprolol, β-blockers/hypotensives; ACTH corticotropin); LH-RH, a neurotransmitter; sulfadiazine and other sulfonamide antibiotics; ribavirin and acyclovir, antiviral agents; chlorambucil and melphalan, nitrogen mustard-type anticancer/antitumor agents; methotrexate and aminopterin, which are folic acid antagonist-type anticancer/antitumor agents; cisplatin-analogue type anticancer/antitumor agents; doxorubicin, daunamycin, dactinomycin and mitomycin C, used in cancer chemotherapy; thioguanine, a purine/pyrimidine antagonist used in cancer treatment; vincristine and vinblastine, anticancer alkaloids; hydroxyurea and DON, anticancer urea derivatives; ~~FSH and HCG, pituitary gonadotropins;~~ N,N'-bis-(dichloracetyl)-1,8-octamethylene diamine, an agent for male fertility inhibition; levorphanol, a narcotic analgesic; and benzestrol and diethylstilbestrol, synthetic estrogens.

Preferred classes of centrally acting drugs for use herein are the central neurotransmitters, steroids, anticancer and antitumor agents, antiviral agents, tranquilizers, memory enhancers and hypotensives. Among the neurotransmitters, there can be mentioned amino acids, such as GABA, glycine, glutamic acid and aspartic acid; catecholamines, such as dopamine, norepinephrine and epinephrine; serotonin and tryptamine; and peptides such as neurotensin, luteinizing hormone-releasing hormone (LHRH), somatostatin, enkephalins such as $met^5$-enkephalin and $leu^5$-enkephalin, endorphins such as γ-, α- and β-endorphins, oxytocin M and vasopressin.

Among the steroids, there can be mentioned antiinflammatory adrenal cortical steroids such as hydrocortisone, betamethasone, cortisone, dexamethasone, flumethasone, fluprednisolone, meprednisone, methyl prednisolone, prednisolone, prednisone, triamcinolone, cortodoxone, fludrocortisone, flurandrenolone acetonide paramethasone and the like; male sex hormones, such as testosterone and methyl testosterone; and female sex hormones, both estrogens and progestins, e.g., ethinyl estradiol, norgestrel, mestranol, norethindrone, ethisterone, estradiol, estriol, estrone, dimethisterone, allylestrenol, cingestol, ethynerone, lynestrenol, norgesterone, norvinisterone, ethynodiol, oxogestone, tigestol, quinestrol and the like. Among the anticancer and antitumor agents, there can be mentioned Ara-AC, pentostatin(2'-deoxycoformycin), Ara-C, 3-deazaguanine, dihydro-5-azacytidine, tiazofurin, sangivamycin, Ara-A, 6-MMPR, PCNU, spiromustine, bisbenzimidazole, L-alanosine, DON, L-ICRF, trimethyl TMM, 5-methyl tetrahydrohomofolic acid, glyoxylic acid sulfonylhydrazone, DACH, SR-2555, SR-2508, desmethylmisonidazole, mitoxantrone, menogarol, aclacinomycin A, phyllanthoside, bactobolin, aphidocolin, homoharringtonine, levonantradol, acivicin, streptozotocin, hydroxyurea, chlorambucil, cyclophosphamide, uracil mustard, melphalan, 5-FUDR, vincristine, vinblastine, cytosine arabinoside, 6-mercaptopurine, thioguanine, 5-azacytidine, methotrexate, adriamycin (doxorubicin), daunomycin, aminopterin, dactinomycin, mitomycin C, and podophyllotoxin derivatives, such as etoposide (VP-16) and teniposide. Among the antiviral agents,

there can be mentioned ribavirin; acyclovir; amantadine; diarylamidines such as 5-amidino-2-(5-amidino-2-benzofuranyl)indole and 4',6-diimidazolino-2-phenylbenzo(b)thiophene; 2-aminoxazoles such as 2-guanidino-4,5-di-n-propyloxazole and 2-guanidino-4,5-diphenyloxazole; benzimidazole analogues such as the syn and anti isomers of 6[[(hydroxyimino)phenyl]methyl]-1-[(1-methylethyl)sulfonyl]-1H-benzimidazol-2-amine; bridgehead C-nucleosides such as 5,7-dimethyl 2-β-D-ribofuranosyl-s-triazole(1,5-a)pyrimidine; glycosides such as 2-deoxy-D-glucose, glucosamine, 2-deoxy-2-fluoro-D-mannose and 6-amino-6-deoxy-D-glucose; phenyl glucoside derivatives such as phenyl-6-chloro-6-deoxy-β-D-glucopyranoside; (S)-9-(2,3-dihydroxypropyl)adenine; 6-azauridine; and 5,6-dichloro-1-β-D-ribofuranosylbenzimidazole. Among the tranquilizers, there can be mentioned benzodiazepine tranquilizers such as diazepam, oxazepam, lorazepam, chlordiazepoxide, flurazepam, bromazepam, clorazepate, nitrazepam and temazepam; hydantoin-type tranquilizers such as phenytoin, ethotoin, mephenytoin; phenothiazine-type tranquilizers such as acetophenazine, carphenazine, fluphenazine, perphenazine and piperacetazine; and others. Among the hypotensives, there can be mentioned clonidine, methyldopa, bethanidine, debrisoquin, hydralazine and guanethidine.

~~By "testicularly acting drug species" as used~~ herein, there is intended any ~~drug which~~ is capable of exerting a ~~useful~~ pharmacological effect when ~~delivered to the testes. Generally speaking, those~~

It will be appreciated that by "dihydropyridine carrier" or "[DHC]", there is intended any nontoxic carrier moiety as hereinbefore defined comprising, containing or including the dihydropyridine nucleus fused to a benzene ring (i.e., a quinoline or isoquinoline ring system) and whether substituted or unsubstituted, the only criterion therefor being capacity for BBB penetration and in vivo oxidation thereof to the corresponding quaternary pyridinium salt carrier $[QC]^+$. As aforesaid, the ionic pyridinium salt drug/carrier prodrug entity $[D\text{-}QC]^+$ which results from such in vivo oxidation is prevented from efflux from the brain while elimination from the general circulation is accelerated. Subsequently, the covalent or equivalent bond coupling the drug species [D] to the quaternary carrier $[QC]^+$ is metabolically cleaved which results in sustained delivery of the drug [D] in the brain and facile elimination of the carrier moiety $[QC]^+$. Such "covalent or equivalent bond" between the drug and the quaternary carrier can be a simple direct chemical bond, e.g., an amide, an ester, or any other like bond, or same can even be comprised of a linking group or function, e.g., a thiazolidine bridge or a peptide linkage, typically necessitated when the drug species is not susceptible to direct chemical coupling to either the dihydropyridine carrier or the quaternary carrier. Nonetheless, the bond in the formulae $[D\text{-}QC]^+$ and [D-DHC] is intended to be and is hereby defined as inclusive of all such alternatives. And the cleavage

of the $[D\text{-}QC]^+$ prodrug to sustainedly deliver the drug species [D] in the brain with concomitant facile elimination of the carrier moiety $[QC]^+$ is characteristically enzymatic cleavage, e.g., by esterase, amidase, cholinesterase, hydrolytic enzyme, or peptidase, albeit any type of in brain cleavage which might result, whether enzymatic, metabolic or otherwise, of course remains within the ambit of this invention. Thus, the drug release rate controlling parameter of the subject pro-prodrugs is imparted simply via the cleavable bonding between drug and carrier, and not by any release rate controlling substituent(s).

The expression "non-toxic pharmaceutically acceptable salts" as used herein generally includes the nontoxic salts of compounds of formula (I), wherein [D] is a centrally acting drug species and [DHC] is the reduced, biooxidizable, blood-brain barrier penetrating form of a dihydropyridine $\rightleftharpoons$ pyridinium salt redox carrier as hereinbefore defined formed with nontoxic, pharmaceutically acceptable inorganic or organic acids HX. For example, the salts include those derived from inorganic acids such as hydrochloric, hydrobromic, sulfuric, sulfamic, phosphoric, nitric and the like; and the salts prepared from organic acids such as acetic, propionic, succinic, glycollic, stearic, lactic, malic, tartaric, citric, ascorbic, pamoic, maleic, hydroxymaleic, phenylacetic, glutamic, benzoic, salicylic, sulfanilic, fumaric, methanesulfonic, toluene-sulfonic and the like.

Examples of redox carriers for use herein include the isoquinolinium acid and alcohol systems below (D represents drug, Z a covalent link) and the corresponding dihydro forms.

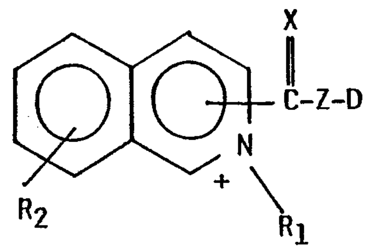

x = $H_2$ or O

Generally preferred dihydropyridine ⇌ pyridinium salt redox carriers for use in the present invention include the following isoquinolinium and quinolinium systems (where D represents the drug), and obviously the corresponding dihydro forms:

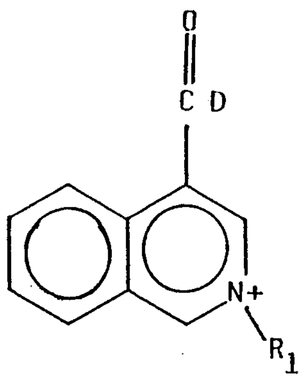

(i)

and

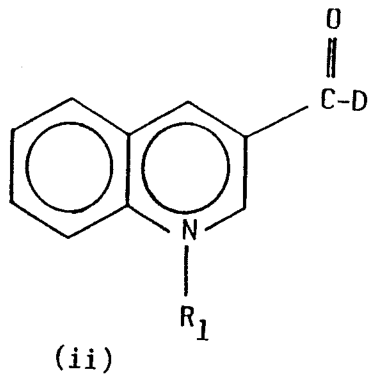

(ii)

in which $R_1$ is $C_1$-$C_7$ alkyl or $C_7$-$C_{10}$ aralkyl.

The corresponding dihydro forms of the foregoing preferred pyridinium salts are depicted below, wherein the identity of the structural variables is as indicated above:

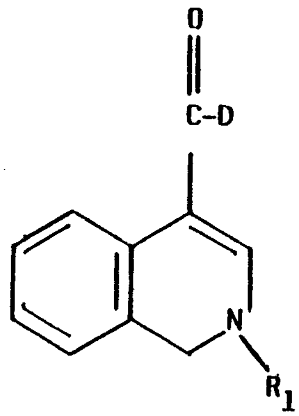

(i)

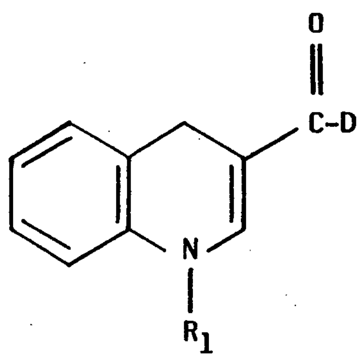

(ii)

Naturally, selection of the particular dihydropyridine ⇌ pyridinium salt redox carrier to be used will depend on the chemical structure of the specific drug involved. And not only should the nature of the functional group which is to be linked to the carrier system be considered in selecting the carrier, but the manner in which the ultimate compound is prepared should be tailored to the presence of any other reactive groups in the molecule.

$R_4$ = H, GABA or valproic acid, but at least one of $R_4$ is:

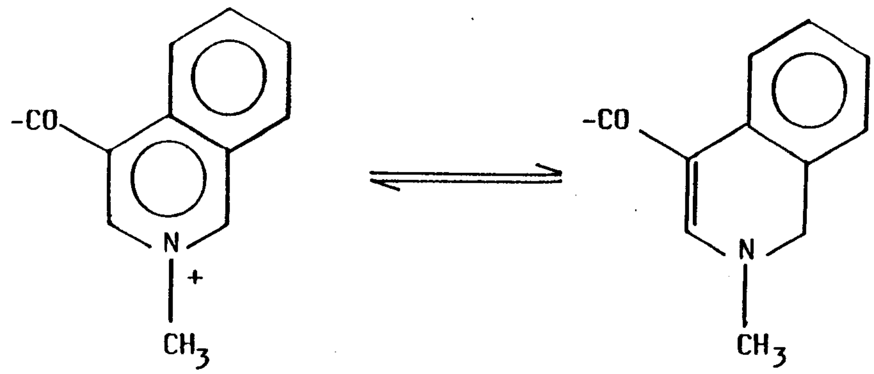

(in 1a and 2a) (in 1 and 2)

$R_4$ can be partially replaced by additional GABA or valproic acid, changing the carrier/drug ratio as necessary. Some of the valproic acid metabolites can be coupled with carriers of the redox type, via the various hydroxy groups formed during the oxidative degradation:

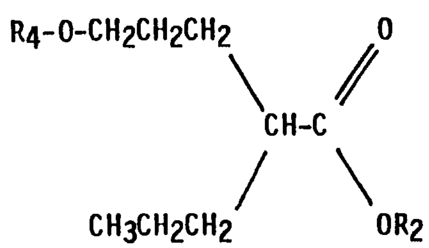

;

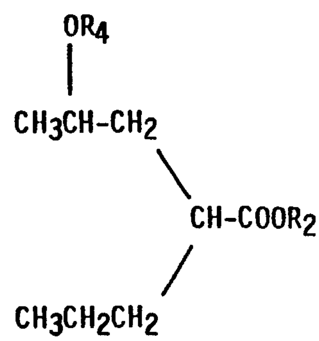

;

3 and 3a

4 and 4a

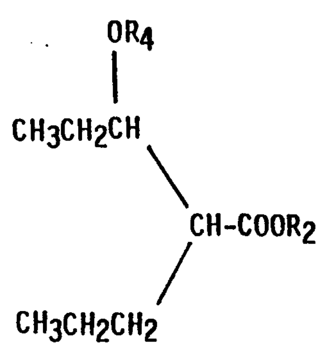

5 and 5a

In another embodiment of like delivery system, applicable for both the GABA and related compounds and for the carboxylic acids, or for any other drug species to be linked to such a carrier, either directly or indirectly, i.e., mediated by a carboxylic acid, e.g., succinic acid, or other linkage, provided is a mono- or poly-substituted nontoxic polyol (such as inositol or sugars) having the trigonelline $\rightleftharpoons$ dihydrotrigonelline system and the compounds to be delivered linked to the same molecule as exemplified by the GABA case (1 $\rightleftharpoons$ 1a) and valproic acid (2 $\rightleftharpoons$ 2a):

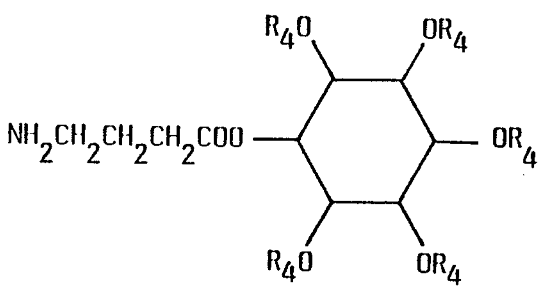

;

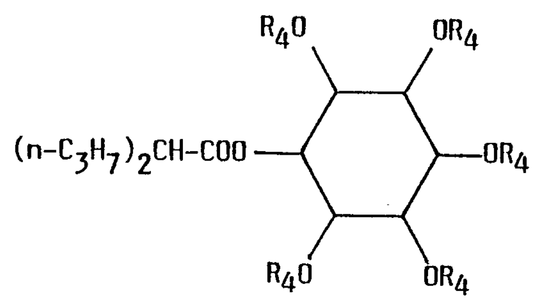

1 $\rightleftharpoons$ 1a

2 $\rightleftharpoons$ 2a

Illustrative examples are the corresponding derivatives of the 5-, 4-, and 3-hydroxy-2-n-propyl pentanoic acid derivatives. Additional carrier systems can also be developed consistent herewith.

Based upon the observation that NADH content is significantly reduced in epileptic and like seizures, the use of the subject redox system (in reduced form) will bias the NAD $\rightleftharpoons$ NADH balance towards NADH during the dihydro carrier → quaternary transformation. Also, the brain-specific delivery of small peptides consistent herewith, e.g., the enkephalins which have been found to initiate epileptic seizures, has led to the design of a variety of long lasting potent antagonists.

And the subject chemical delivery system is also useful for the delivery of other anticonvulsants in a sustained, brain-specific fashion, e.g., the benzodiazepines and hydantoins, and those compounds, like apomorphine, which are useful in the treatment of photosensitive epilepsy.

It will of course be appreciated in the immediately above regard that the drug treatment of epilepsy has always posed formidable problems. There are many different anticonvulsants available, some more specific for different types of seizures. Indeed, there exist a wide variety of opinions as to which is the most suitable drug for any particular type of seizure, and drug mixtures are typically employed. An inevitable result of the traditional therapy is the development of chronic toxicity, but such result is conspicuously avoided according to the present invention.

It will be appreciated that the desired therapeutic effects of all antiepileptic agents investigated, as well as their undesired toxic effects, reflect a statistically significant correlation with the drug levels in plasma. This correlation is based upon a close relationship between the drug concentrations in plasma and brain tissue. In contrast, a primary attribute of this invention is to enable attainment of high and sustained brain levels of the selected active agents, essentially against the plasma-brain concentration gradient and independent of the drug concentration in the blood.

GABA and related compounds are logical candidates. It has been shown that GABA neuron function is impaired in at least certain types of human epilepsy. Animal studies also showed that seizures are induced by reduction of GABA neuron function to a critical degree by (1) inhibition of GABA synthesis, (2) blockade of GABA receptors or (3) inhibition of GABA-receptor mediated ionic events. In addition, enhancement of GABA synaptic activity (by direct receptor stimulation or by increasing GABA levels in the synapse) has a potent and wide spectrum anticonvulsant effect. These findings foreshadowed that an enhanced and sustained GABA brain delivery or a brain-specific delivery in a sustained manner of a good GABA-agonist would be efficacious in different forms of epilepsy. It is well known that GABA itself, when administered systematically, does not penetrate the normal blood-brain barrier to any significant extent. Among the potential sites at which drugs may act to influence GABA-mediated synaptic function, the first target is to effect the BBB transfer of GABA via a redox delivery system. The second

main target is to effect the catabolism of GABA. This invention, accordingly, specifically provides for the efficacious delivery of the GABA-T inhibitors, γ-vinyl and γ-acetylene-GABA, but the delivery of valproic acid, specifically to the brain and without requiring high circulating blood levels, is also envisaged. In order to achieve the required activity, sodium valproate must have a relatively high, 50-100 μg/ml, level in the blood. The value of valproic acid is well established in most types of epilepsy. It is evident that valproic acid produces significant increases in both brain and synaptosomal GABA concentrations. Valproic acid itself undergoes extensive metabolism.

In capsule summary, the present invention provides for the significantly improved treatment of epilepsy, and concomitant reduction in toxicity of a number of antiepileptic drug species currently in use. And made available to the brain is a variety of important compounds, such as GABA and a wealth of GABA-ergic agents.

It will be apparent from the foregoing discussion of specific drug-carrier combinations that a wide variety of synthetic approaches can be utilized, depending on the chemical nature of the selected drug. Various illustrative synthetic schemes as applied to specific centrally acting drugs in accord with this invention are set forth below in the section entitled "Illustrative Synthetic Methods". While the sequence of reaction steps can be varied in many cases, in general, the final step (except in the case of optional

salt formation) will be reduction of a pyridinium compound of formula (II) to the corresponding dihydropyridine compound of formula (I). The reduction is usually conducted at a temperature from about -10°C to room temperature, for a period of time from about 10 mins to 2 hrs, conveniently at atmospheric pressure. Typically, a large excess of reducing agent is employed, e.g., a 1:5 molar ratio of reducing agent to starting $[D\text{-}QC]^+$ compound. The process is conducted in the presence of a suitable reducing agent, preferably an alkali metal dithionite such as sodium dithionite or an alkali metal borohydride such as sodium borohydride or lithium aluminum borohydride, in a suitable solvent. Sodium dithionite reduction is conveniently carried out in an aqueous solution; the dihydro product [D-DHC] is usually insoluble in water and thus can be readily separated from the reaction medium. In the case of sodium borohydride reduction, an organic reaction medium is employed, e.g., a lower alkanol such as methanol, an aqueous alkanol or other protic solvent.

Suitable nontoxic pharmaceutically acceptable carriers (i.e. diluents or vehicles) for use with the topic compounds [D-DHC], e.g., those less toxic than the target drug species themselves, will be apparent to those skilled in this art. Compare, for example, Remington's Pharmaceutical Sciences, 4th Edition (1970). Obviously, the choice of suitable carriers (i.e. diluents or vehicles) will depend upon the exact nature of the particular dosage form selected, as well as upon the identity of the active drug species [D]. The therapeutic dosage ranges for administration of the compounds according to this invention will generally be the same as, or less than, those characteristically used in this art for administration of the known drug species [D], per se. Naturally, such therapeutic dosage ranges will vary with the size of the patient, the condition for which the [D-DHC] compound is administered, the particular dosage form employed, and the like. The quantity of given dosage form needed to deliver the desired dose of [D] will of course depend upon the concentration of [D-DHC] in any given pharmaceutical composition/dosage form thereof.

The ability of the topic compounds to cross the BBB and to be "locked into" the brain allows administration of the drug in a site-specific manner. A combination of the present dihydropyridine ⇌ pyridinium salt redox system with a sustained release system will further enhance this site-specificity. Thus, a preferred embodiment of the invention comprises formulating the [D-DHC] compound or the salt of a [D-DHC] compound

utilizing a sustained release carrier (i.e. diluent or vehicle) system and/or route of administration capable of slowly releasing the chemical, e.g., sustained release tablets and capsules for oral administration; subcutaneous injection, or implantation of drugs in solid pellet form (for example, distributed in a biodegradable polymer); intramuscular injection of the compound in solution in oil or suspended in a repository vehicle; a transdermal delivery device or form such as an ointment to be applied locally to the desired site (when the drug is susceptible of delivery through the skin) and the like. The rate of release of compound from the sustained release system should be comparable to the rate of in vivo oxidation of the dihydro form of the redox system in order to achieve the greatest degree of enhancement of specificity.

## Illustrative Synthetic Methods

### I. Methods for Derivatizing $-NH_2$ or -NH- Functions in Drugs

#### Method A

The drug is reacted with 3-quinolinecarboxylic acid or its acid chloride or anhydride (in the case of the acid, in the presence of a suitable dehydrating agent such as dicyclohexylcarbodiimide), in an appropriate organic solvent, to afford the corresponding amide. The amide is then quaternized, typically by treatment with methyl iodide in a suitable organic solvent, to afford the quaternary derivative $[D\text{-}QC]^+$, which is then reduced by treatment with sodium dithionite or sodium borohydride as generally described hereinabove to afford the desired compound [D-DHC].

The representative drugs listed below may be derivatized in this manner to the corresponding $[D\text{-}QC]^+$ and [D-DHC] compounds.

Starting Material | [D-QC]$^+$ | [D-DHC]

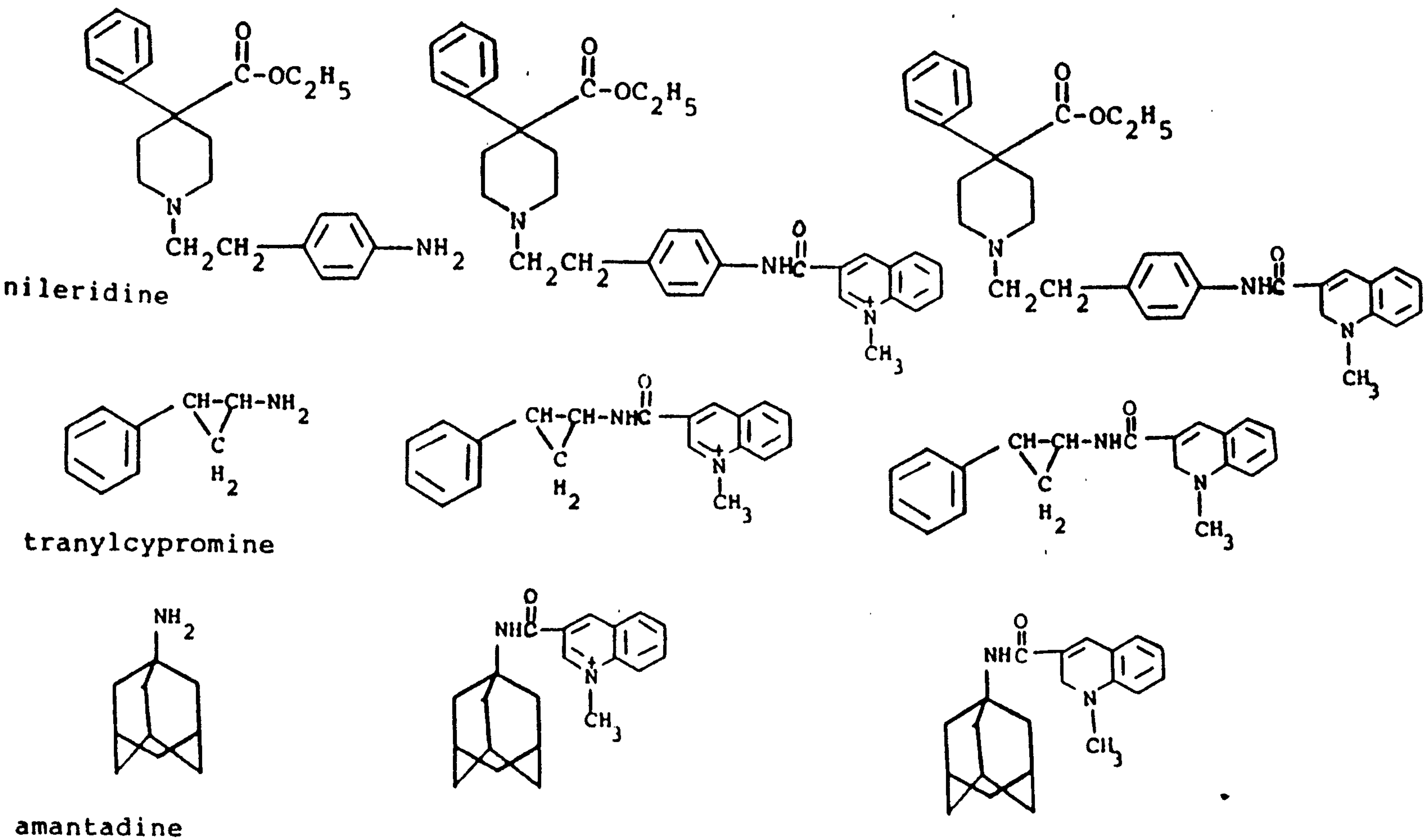

anileridine

tranylcypromine

amantadine

| Starting Material | [D-QC]$^+$ | [D-DHC] |
|---|---|---|
| 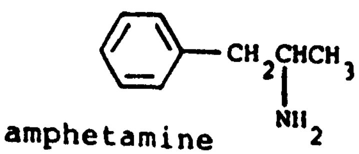 amphetamine | 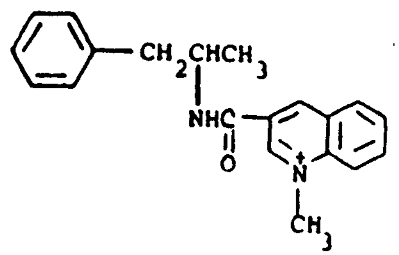 | 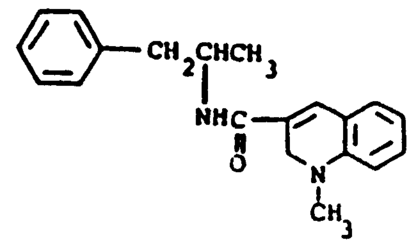 |
| 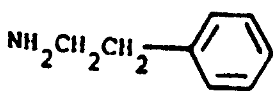 phenylethylamine | 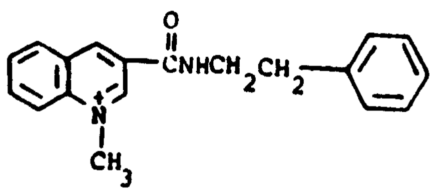 | 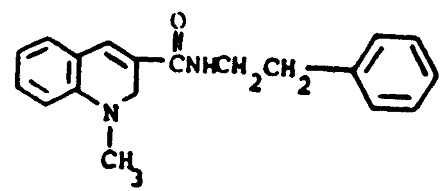 |
| 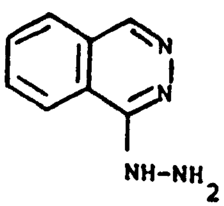 hydralazine | 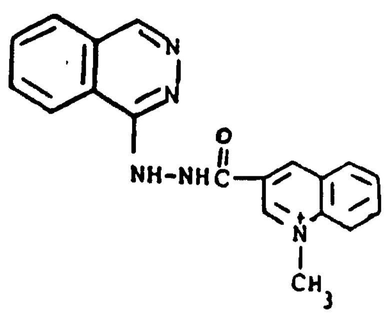 | 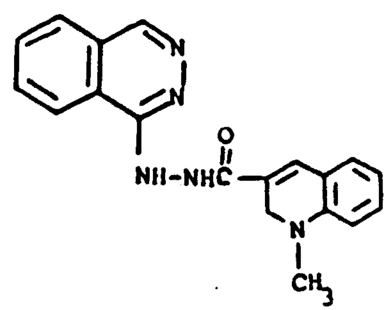 |

| Starting Material | [D-QC]$^+$ | [D-DHC] |
|---|---|---|
| 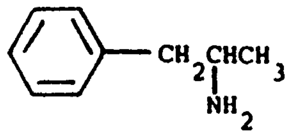 d-isomer dextroamphetamine | 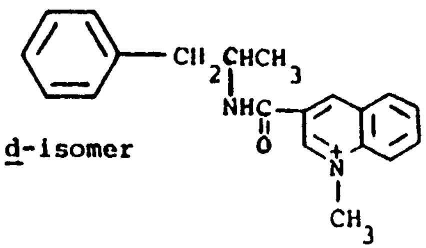 d-isomer | 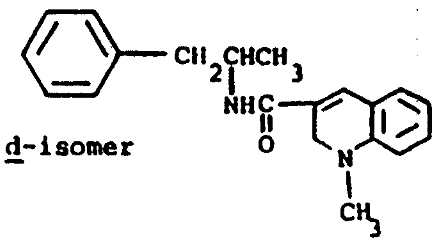 d-isomer |
| 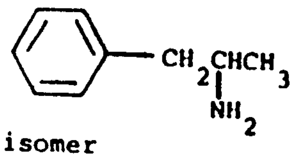 l-isomer levamphetamine | 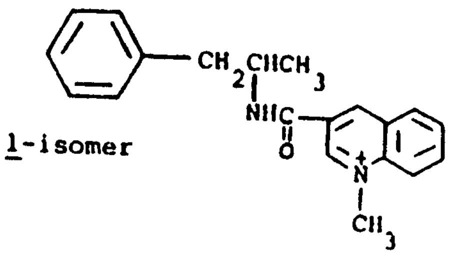 l-isomer | 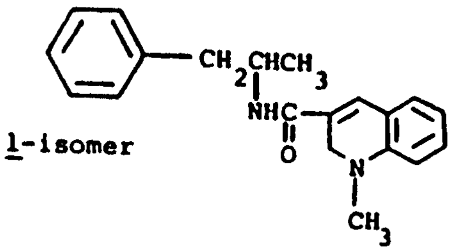 l-isomer |
| 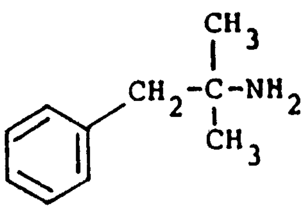 phentermine | 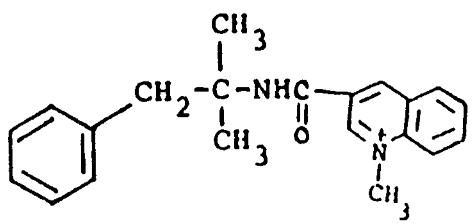 | 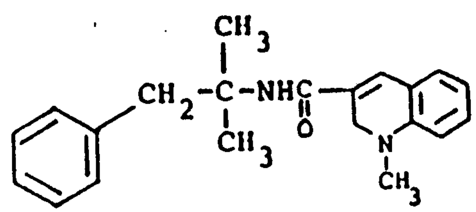 |

The following drugs may be derivatized in similar manner:

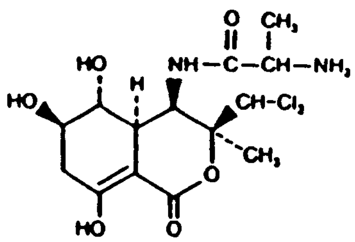

bactobolin

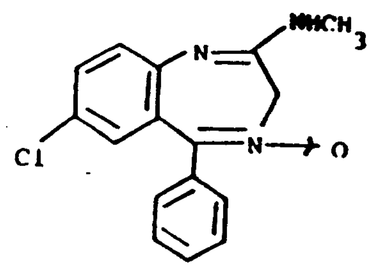

chlordiazepoxide

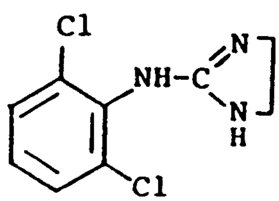

clonidine

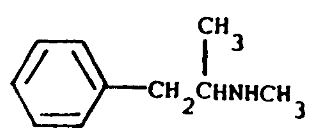

methamphetamine

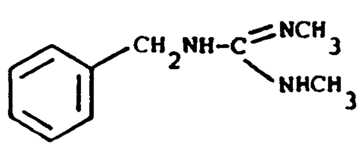

bethanidine

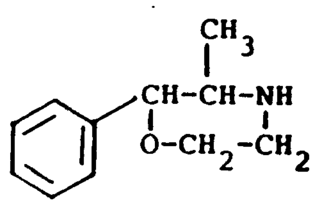

phenmetrazine

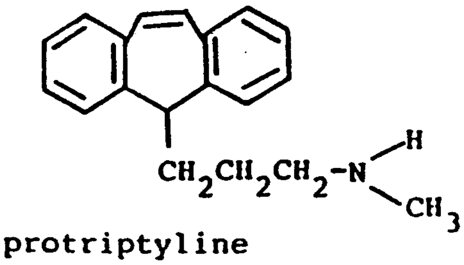

protriptyline

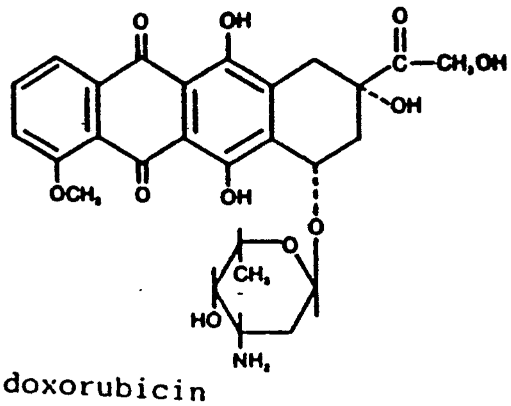

doxorubicin

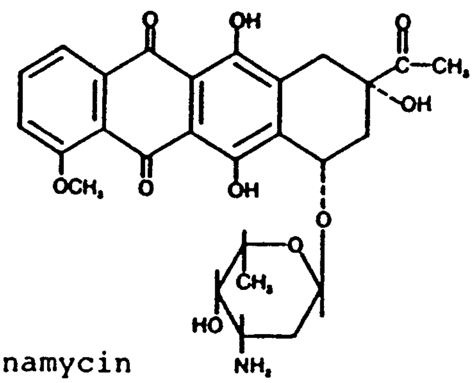

daunamycin

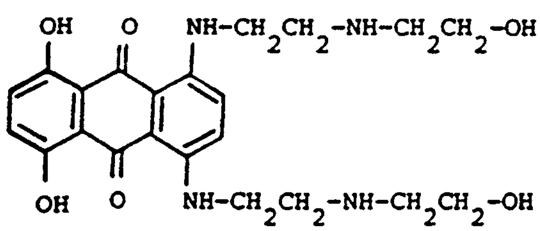

mitoxantrone

Furthermore, Method A may be combined with Methods B, C or D below.

Method B

This is a variation of Method A used when the drug contains a -COOH function which is to be protected.

The drug is first converted to the corresponding ethyl ester by conventional esterification techniques. That ester is then used as the starting material and Method A is repeated.

Obviously, other esters may be similarly prepared in the first step by use of other esterifying agents.

The following representative drugs ~~listed below~~ may be derivatized in this manner to the corresponding $[D-QC]^+$ and [D-DHC] compounds:

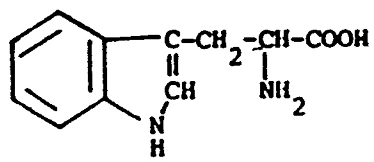

tryptophan

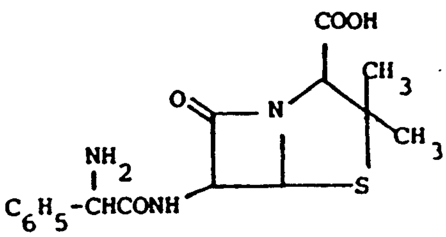

ampicillin

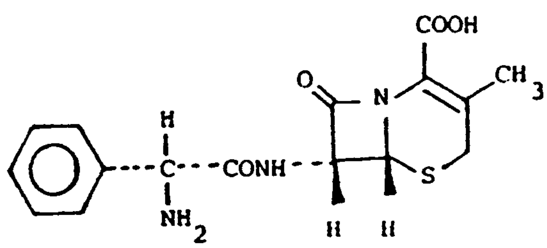

cephalexin

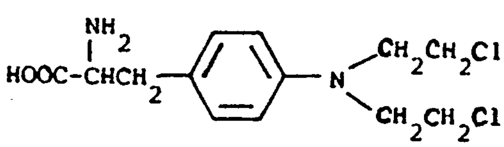

melphalan

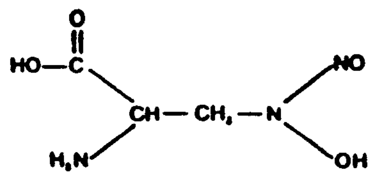

L-alanosine

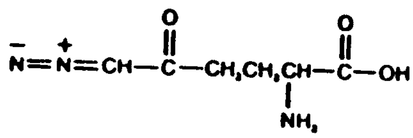

DON

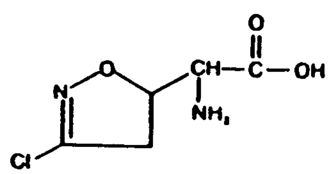

acivicin

$NH_2-CH_2CH_2CH_2COOH$

GABA

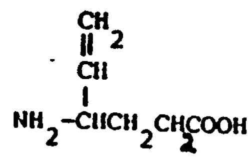

γ-vinyl GABA

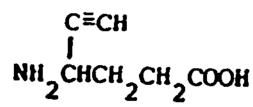

γ-acetylenic GABA

Method C

This is a variation of Method A used when the drug contains one or more OH functions which are to be protected.

The drug is first reacted with excess trimethylacetyl chloride to convert the hydroxy group(s) to pivalyloxy group(s). (This process is generally conducted in the presence of a base; however, strongly acid conditions are used if an amine function is present.) That protected derivative is then used as the starting material and subjected to Method A. Alternatively, the first two steps may be reversed, i.e. the drug may be first converted to the ~~nicotin~~amide, which may then be reacted with trimethylacetyl chloride to form the protected ~~nicotin~~amide.

Various other hydroxy protecting groups may be introduced in similar fashion.

The following representative drugs ~~listed below~~ may be derivatized in this manner to the corresponding $[D\text{-}QC]^+$ and [D-DHC] compounds:

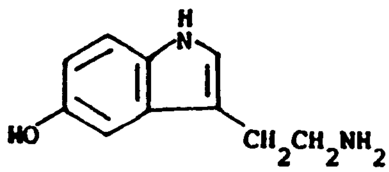

serotonin

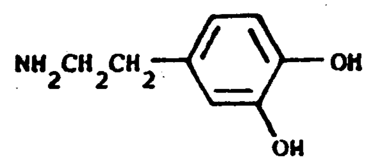

dopamine

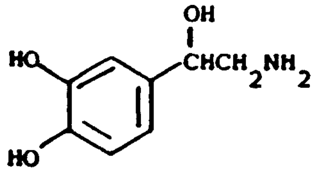

norepinephrine

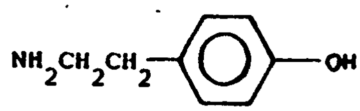

tyramine

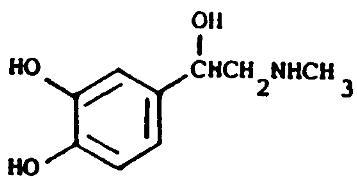

epinephrine

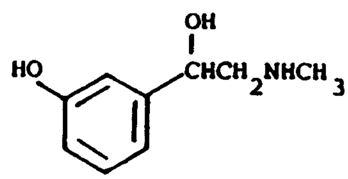

phenylephrine

Method D

This variation of Method A can be used when the drug contains one or more OH and COOH functions which are to be protected. The protecting groups, typically the ethyl ester and pivalyloxy groups, are introduced as described in Methods B and C, in the sequence considered most convenient. The amine function is derivatized according to Method A.

The following representative drugs may be derivatized in this manner to the corresponding $[D\text{-}QC]^+$ and [D-DHC] compounds:

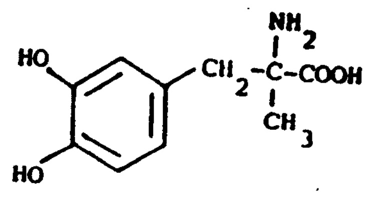

methyldopa

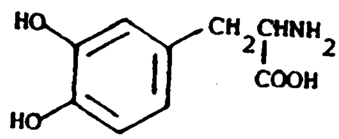

levodopa

Method E

This method is of particular use when the -NH- function is part of an amide or imide or a very low pKa primary or secondary amine.

The drug is first reacted with an aldehyde [e.g. formaldehyde, benzaldehyde, acetaldehyde or chloral ($Cl_3CCHO$)]; for example, in the case of chloral, one converts the -NH- function to a

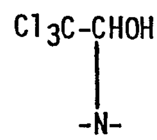

function and thus forms a suitable bridging group. The resultant compound is then reacted with 3-quinolinecarboxylic acid in the presence of a suitable dehydrating agent, or with the acid chloride or anhydride, to form the corresponding ester of the partial formula

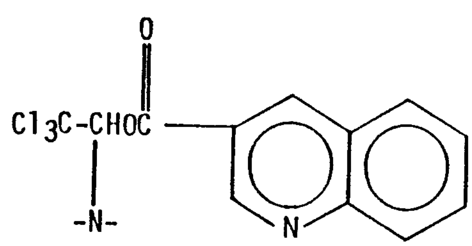

The resultant intermediate is then quaternized and reduced as in Method A.

The following representative starting drugs may be derivatized in this manner to the corresponding $[D\text{-}QC]^+$ and [D-DHC] compounds:

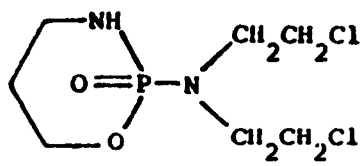

cyclophosphamide

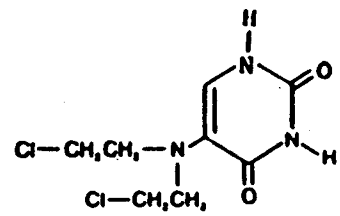

uracil mustard

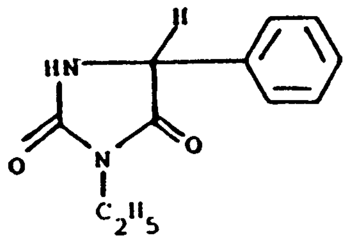

ethotoin

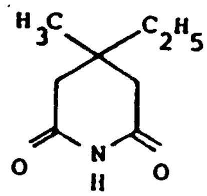

bemegride

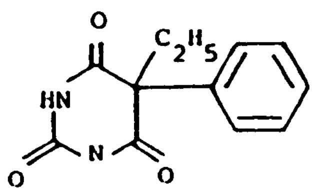

phenobarbital

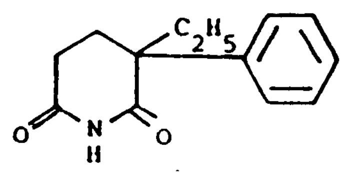

glutethimide

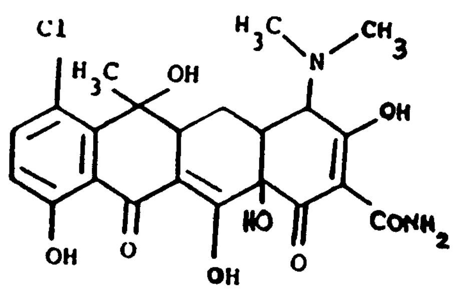

chlortetracycline

## II. Methods for Derivatizing -OH and -SH Functions in Drugs

### Method F

The drug is reacted with 3-quinolinecarboxylic acid or its acid chloride or anhydride (in the case of the acid, in the presence of a suitable dehydrating agent such as dicyclohexylcarbodiimide), in an appropriate organic solvent, to afford the corresponding ester. The ester is then quaternized and subsequently reduced as described above in Method A. When the drug contains more than one reactive hydroxyl or thiol functions, reaction conditions may be varied so that more than one hydroxyl or thiol function will be converted to ester groupings.

The representative drugs listed below may be derivatized in this manner to the corresponding $[D\text{-}QC]^+$ and [D-DHC] compounds.

Starting Material | [D-QC]$^+$ | [D-DHC]

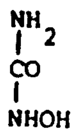

hydroxyurea

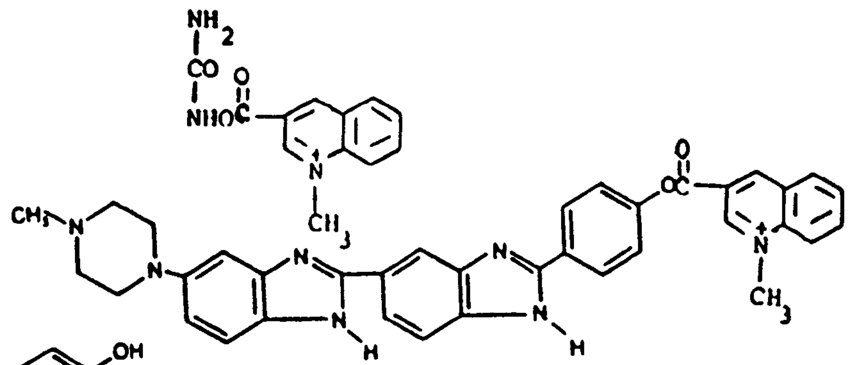

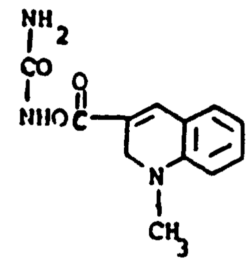

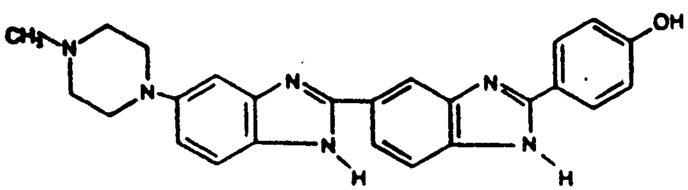

bisbenzimidazole

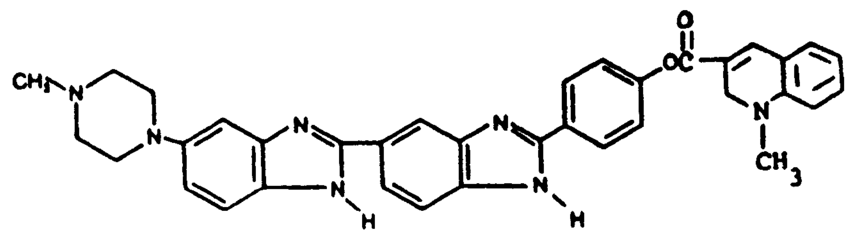

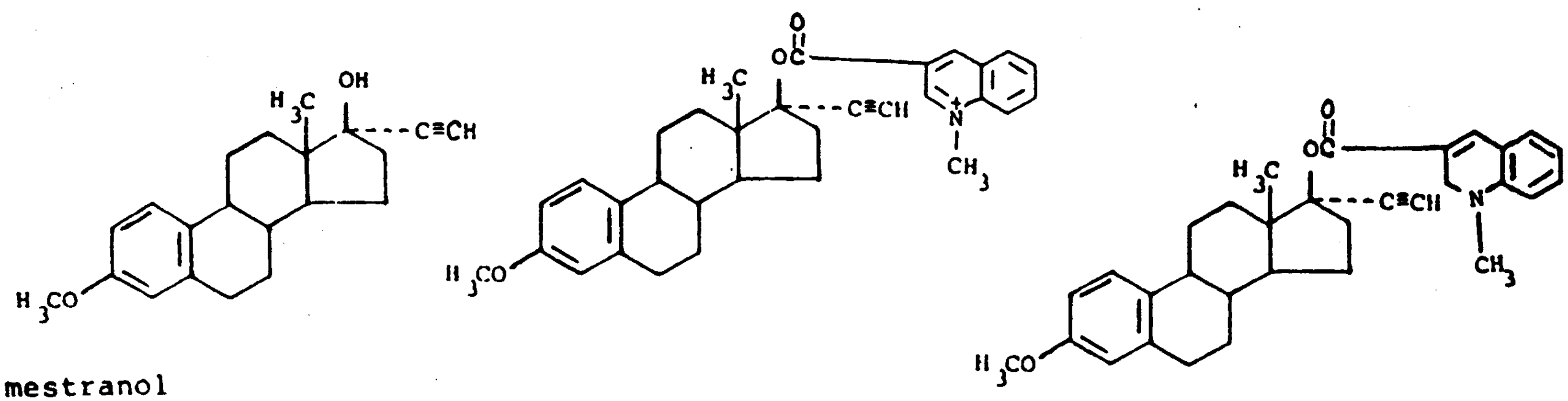

mestranol

Starting Material | $[D-QC]^+$ | [D-DHC]

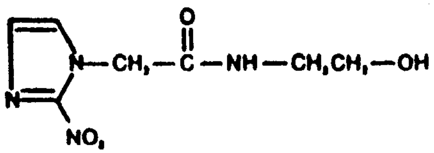

SR-2508

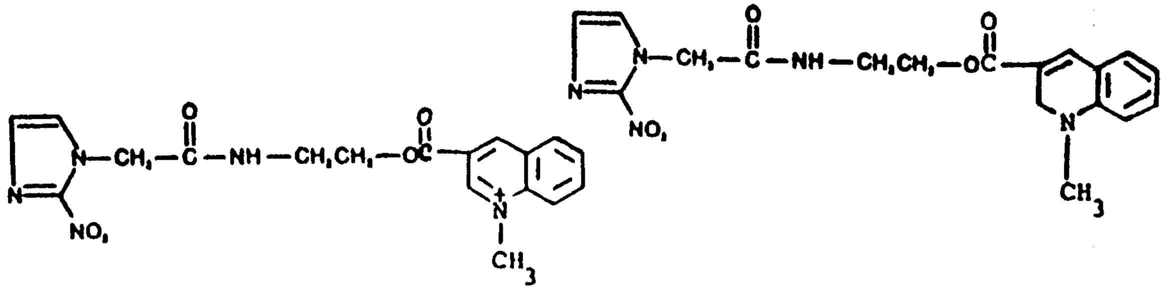

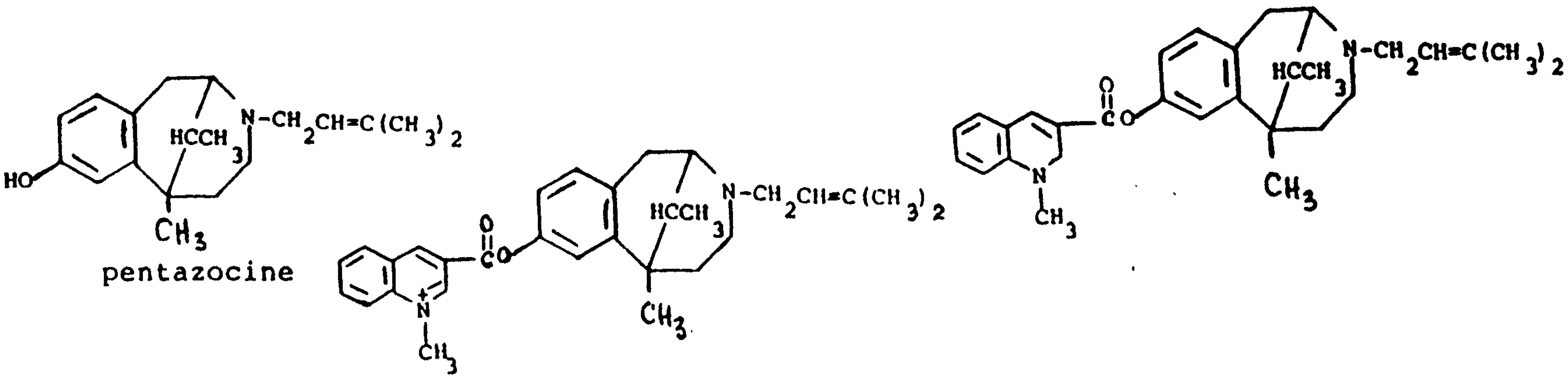

pentazocine

Starting Material | $[D\text{-}QC]^+$ | [D-DHC]

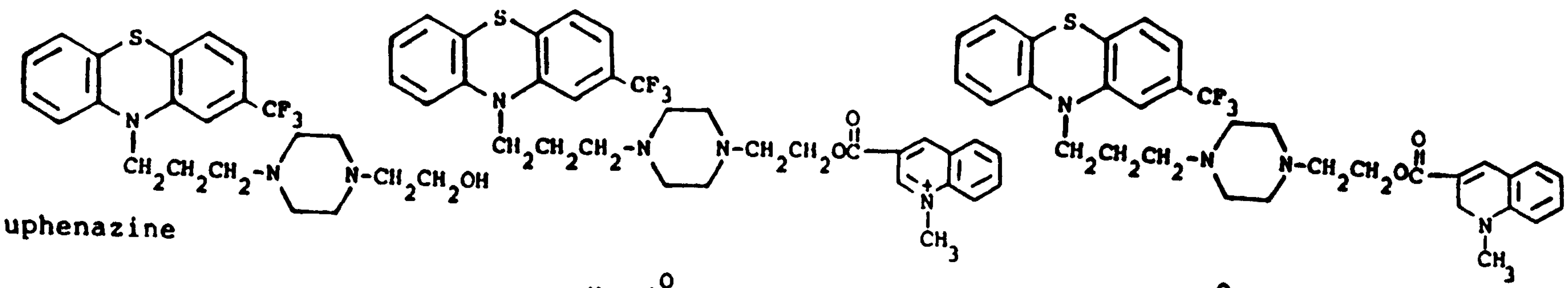

fluphenazine

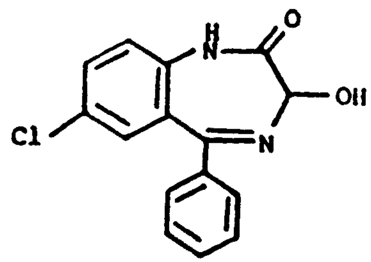

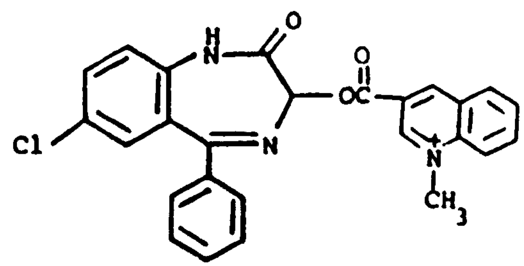

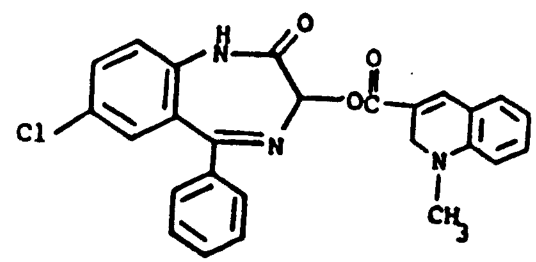

oxazepam

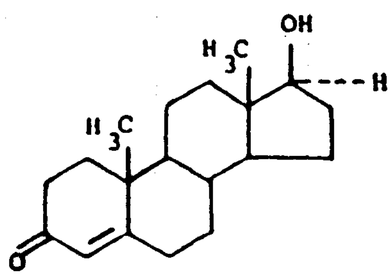

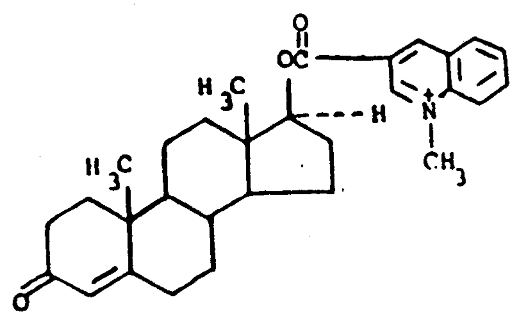

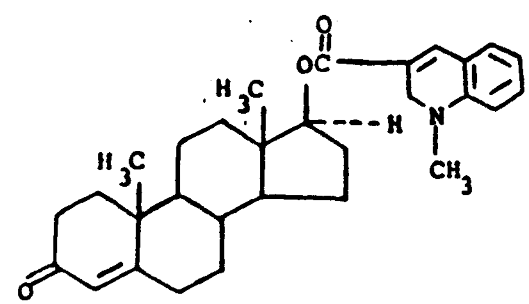

testosterone

The following drugs may be derivatized in similar manner:

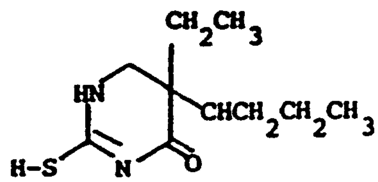

pentothal
(thiopental)

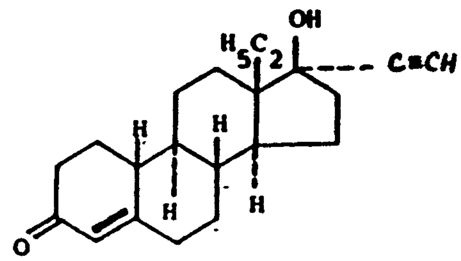

norgestrel

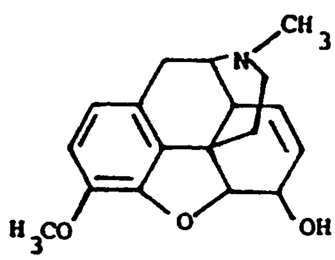

codeine

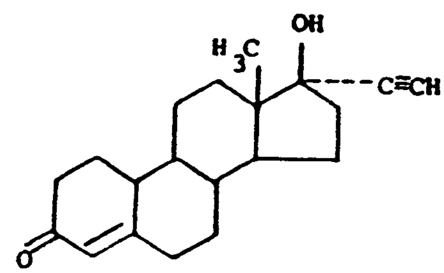

norethindrone

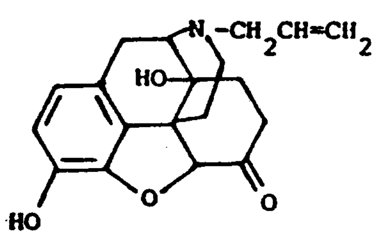

naloxone

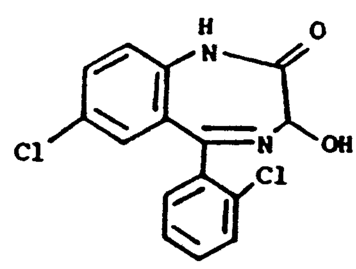

lorazepam

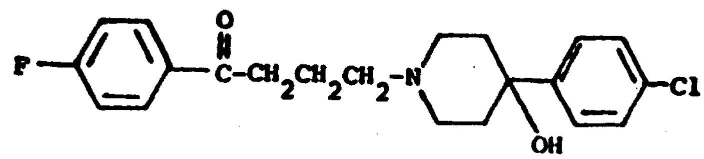

haloperidol

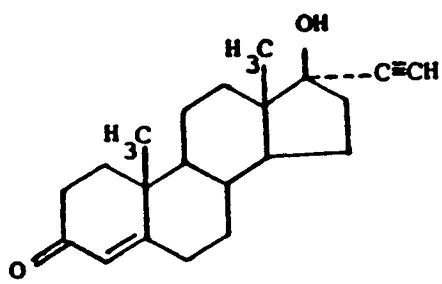

ethisterone

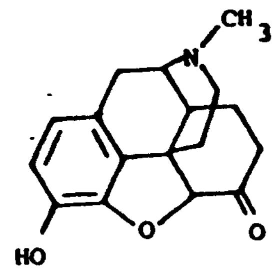

hydromorphone

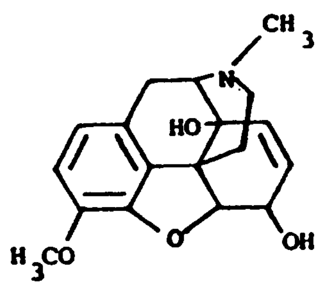

oxycodone

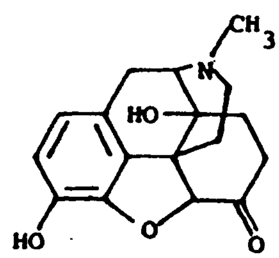

oxymorphone

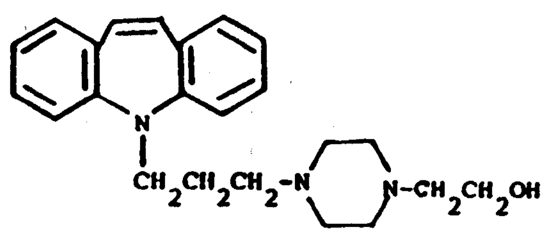

opipramol

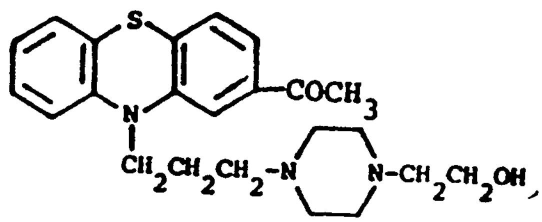

acetophenazine

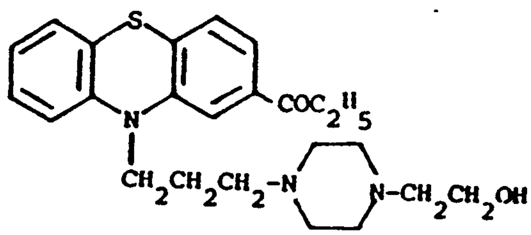

carphenazine

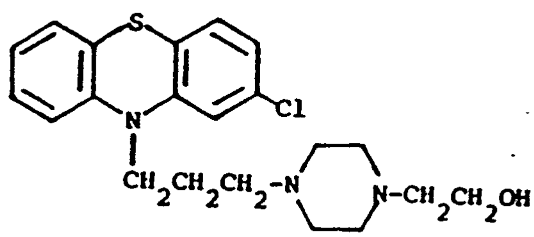

perphenazine

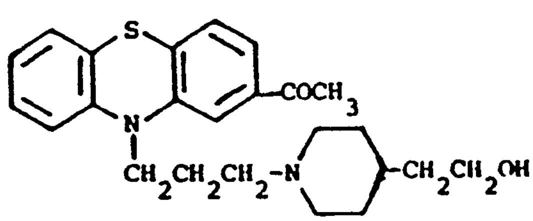

piperacetazine

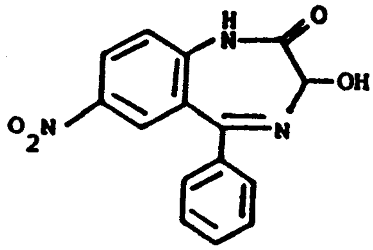

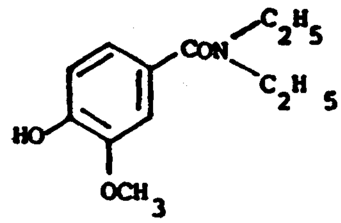

ethamivan

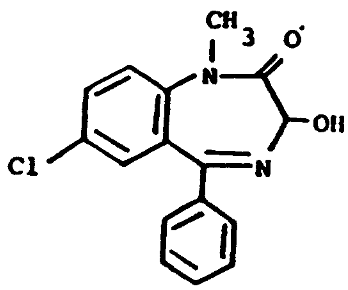

temazepam

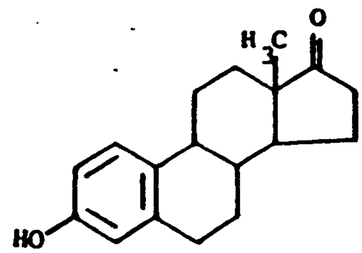

estrone

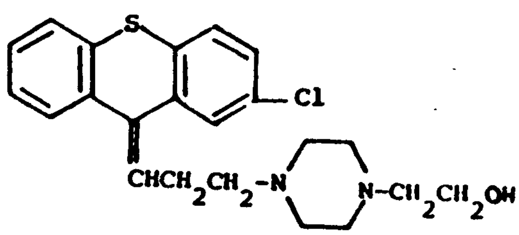

clopenthixol

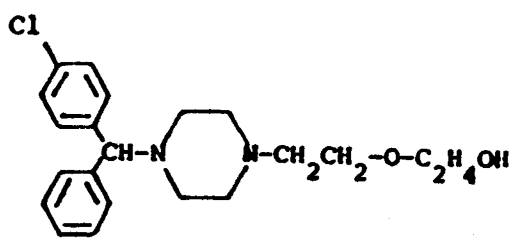

hydroxyzine

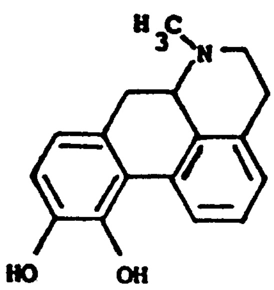

apomorphine

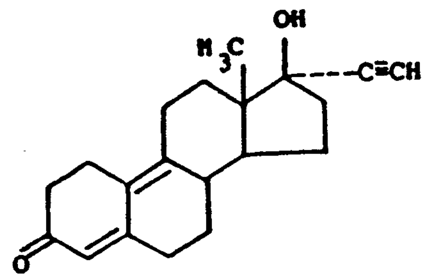

ethynerone

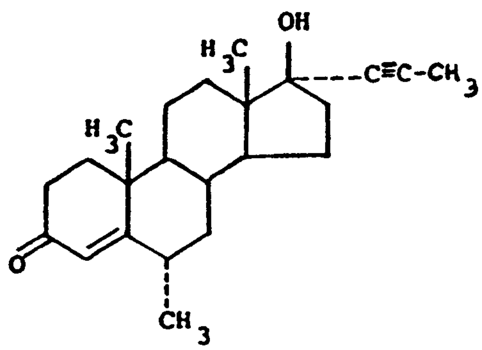

dimethisterone

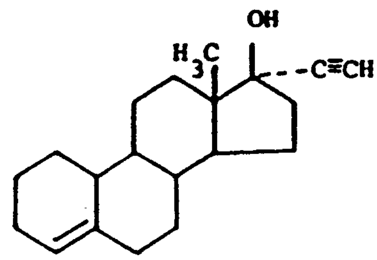

lynestrenol

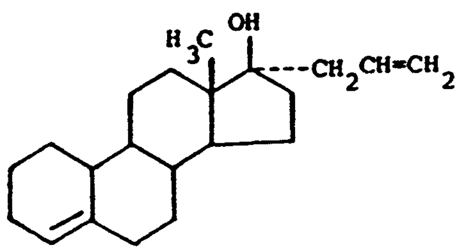

allylestrenol

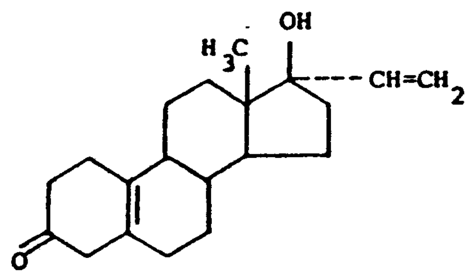

norgesterone

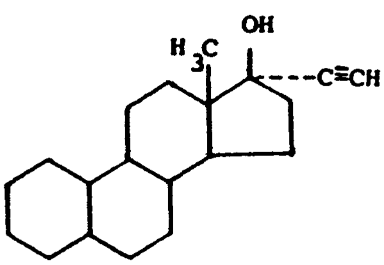

cingestol

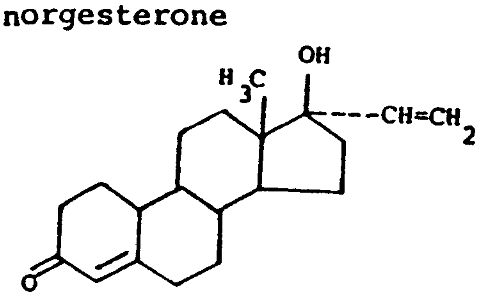

norvinisterone

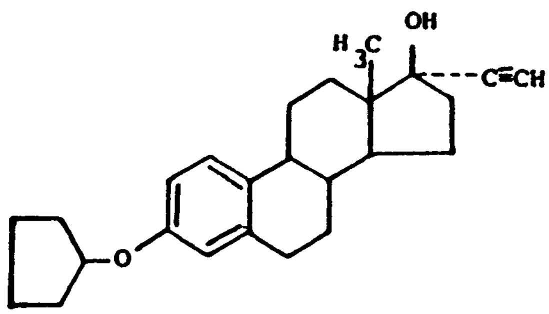

quinestrol

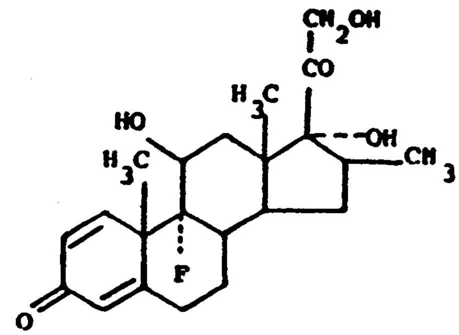

betamethasone

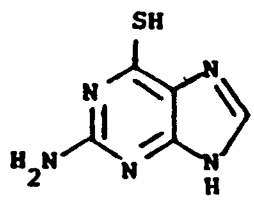

thioguanine

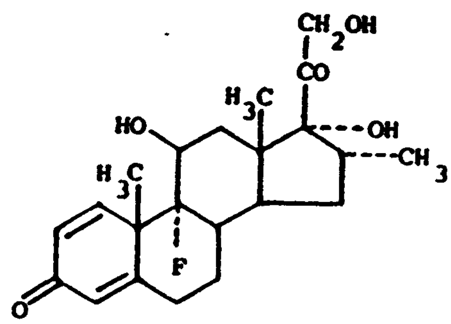

dexamethasone

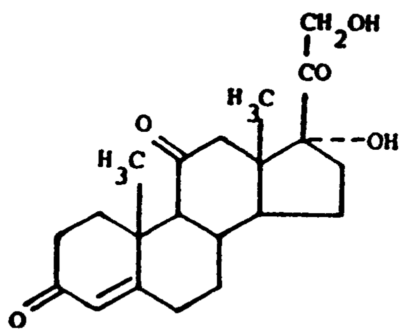

cortisone

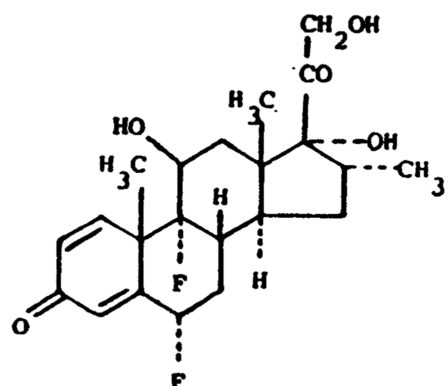

flumethasone

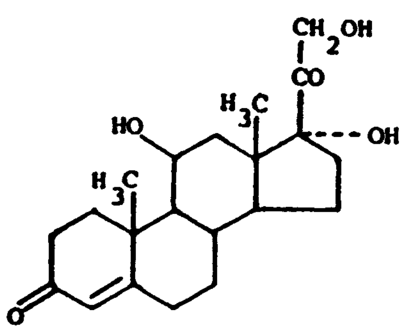

hydrocortisone

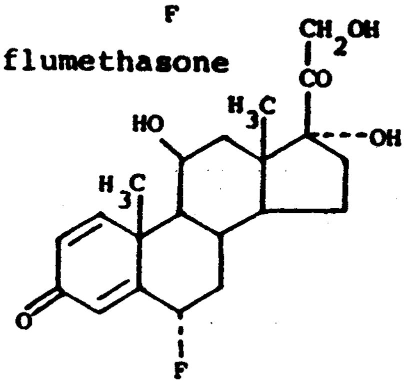

fluprednisolone

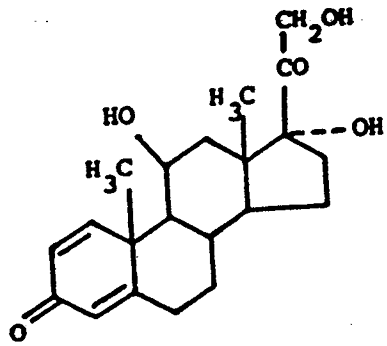

prednisolone

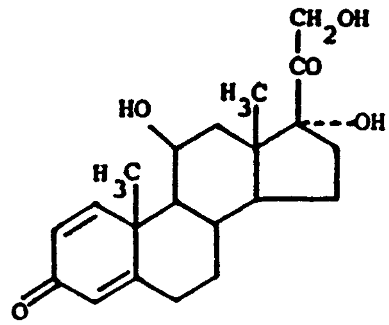

methyl prednisolone

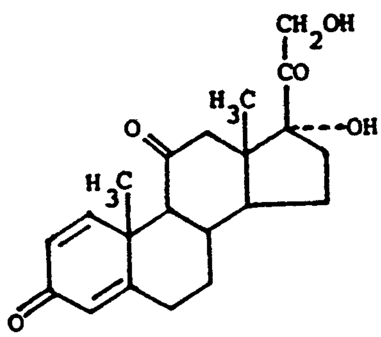

prednisone

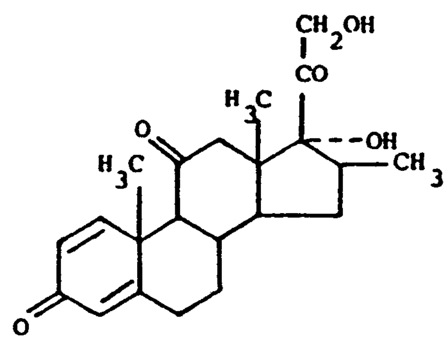

meprednisone

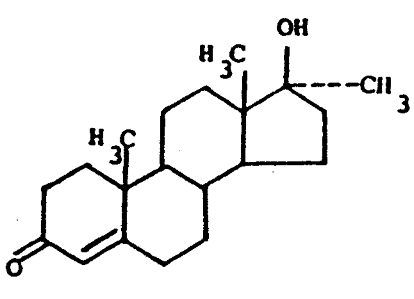

methyl testosterone

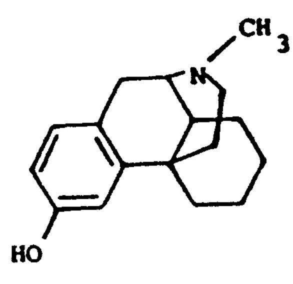

levorphanol

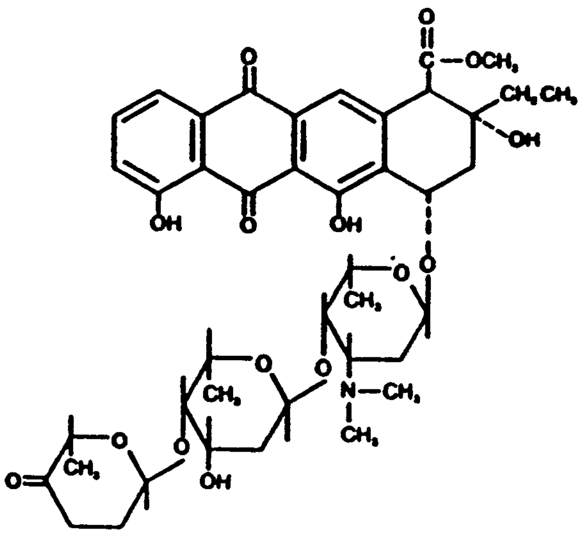

aclacinomycin A

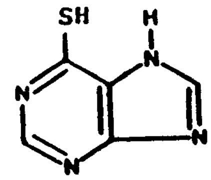

6-mercaptopurine

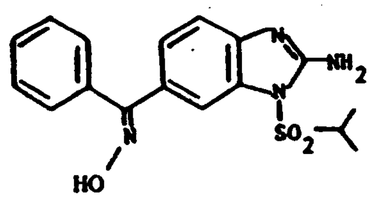

anti-6-[[(hydroxyimino)-phenyl]methyl]-1-[(1-methylethyl)sulfonyl]-1H-benzimidazol-2-amine

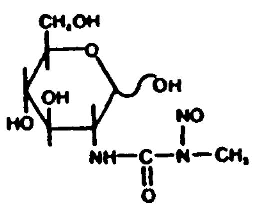

streptozotocin

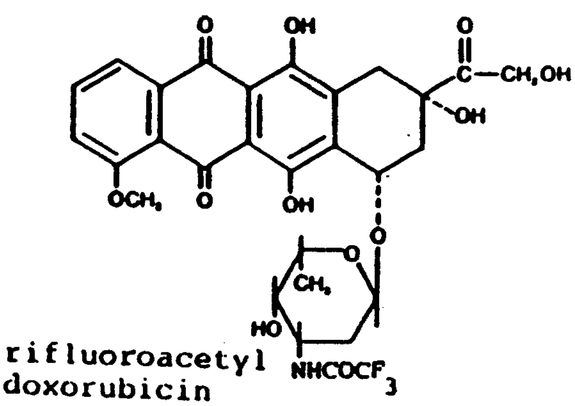

trifluoroacetyl doxorubicin

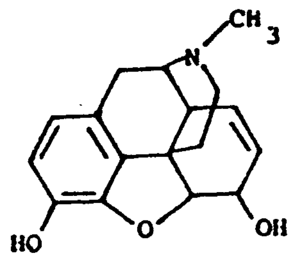

morphine

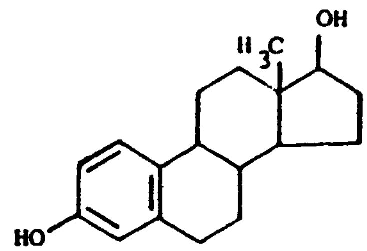

estradiol

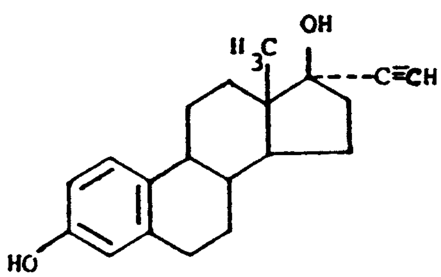

ethinyl estradiol

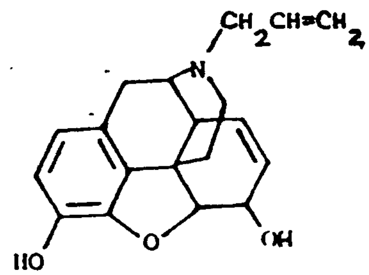

nalorphine

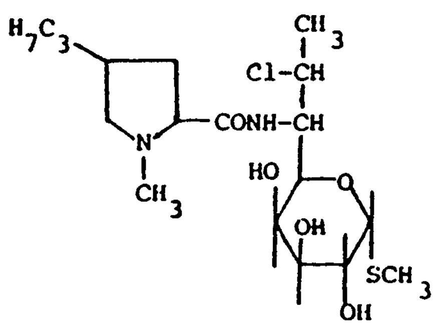

clindamycin

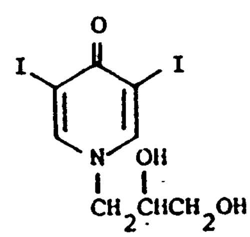

iopydol

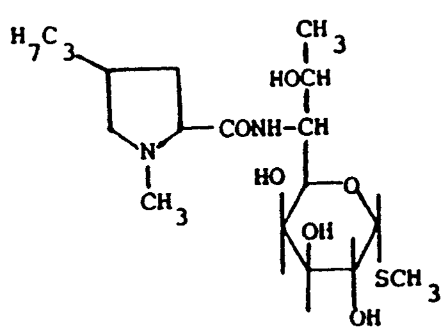

lincomycin

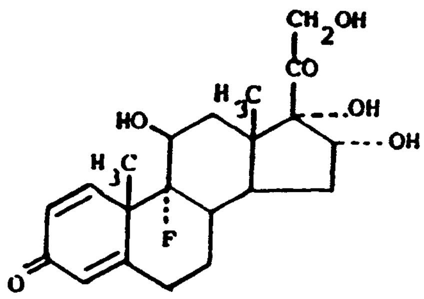

triamcinolone

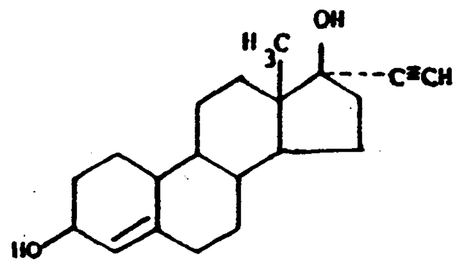

ethynodiol

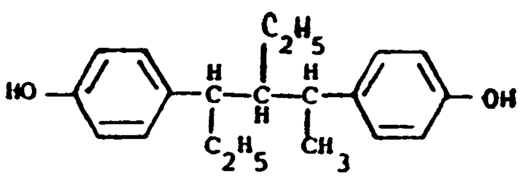

benzestrol

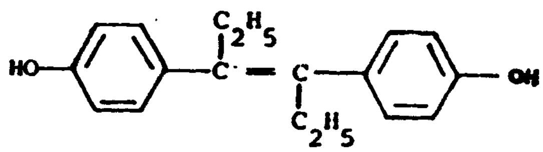

diethylstilbestrol

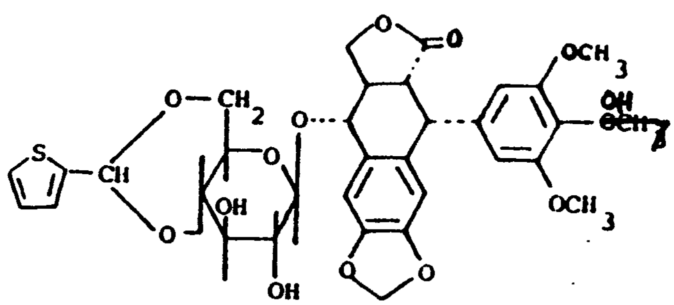

teniposide

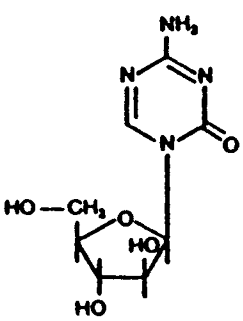

Ara-AC

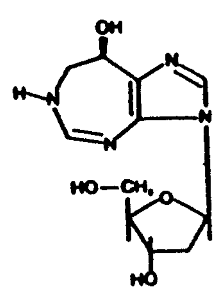

pentostatin
(2'-deoxycoformycin)

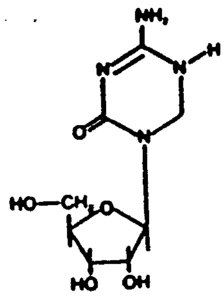

dihydro-5-
azacytidine

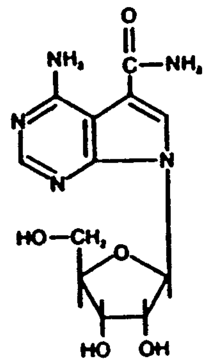

sangivamycin

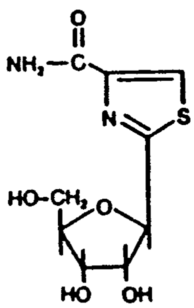

tiazofurin

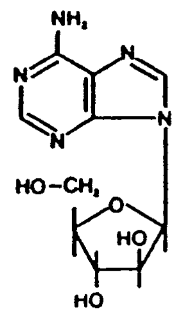

Ara-A

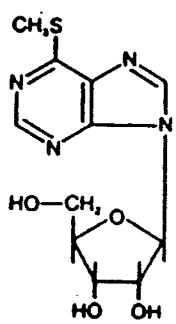

6-MMPR

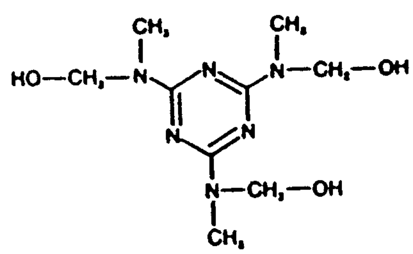

trimethyl TMM

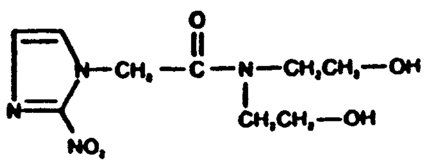

SR-2555

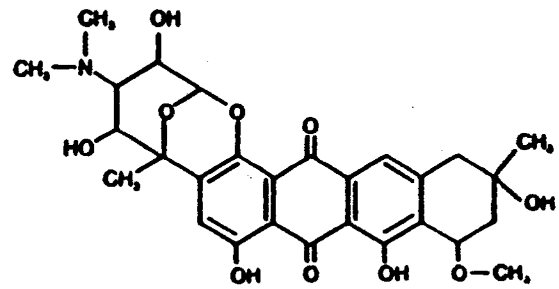

menogarol

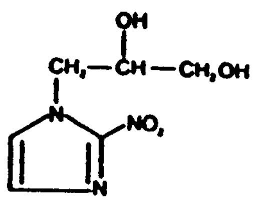

desmethylisonidazole

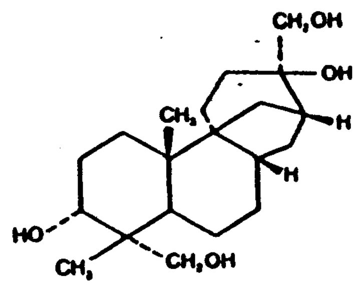

aphidicolin

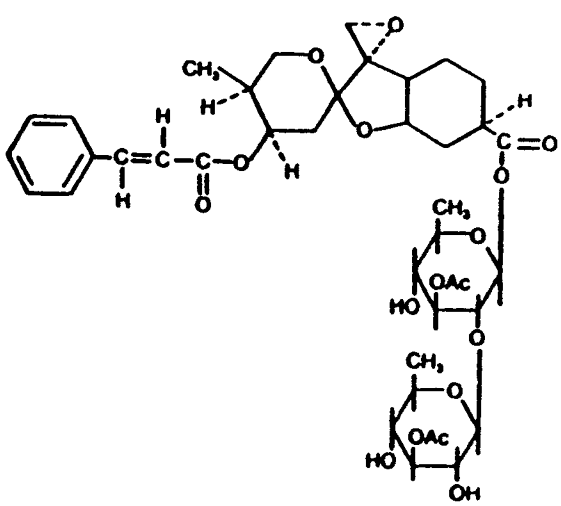

phyllanthoside

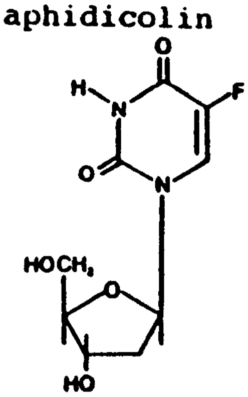

5-FUDR

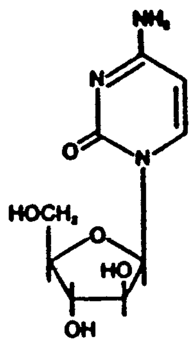

cytosine arabinoside

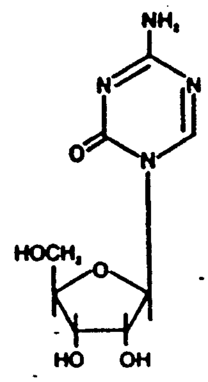

5-azacytidine

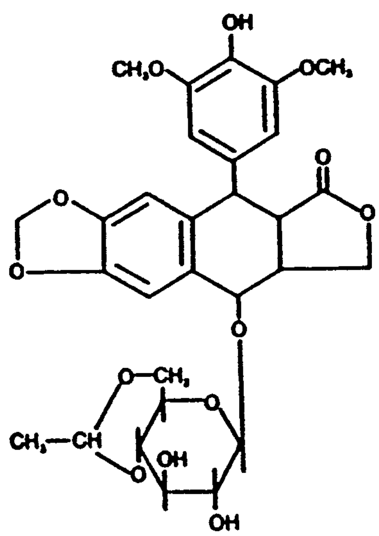

VP-16 (etoposide)

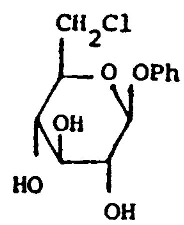

phenyl-6-chloro-
6-deoxy-β-D-
glucopyranoside

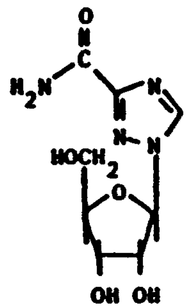

ribavirin

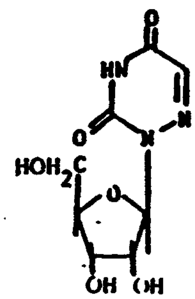

6-azauridine

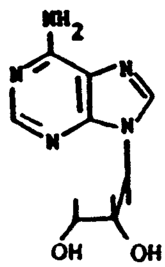

(S)-9-(2,3-dihydroxypropyl)-adenine

Similarly, Method F may be combined with Method G below.

Method G

This is a variation of Method F used when the drug contains a -COOH function which is to be protected.

The drug is first converted to the corresponding ethyl ester by conventional esterification techniques. That ester is then used as the starting material and Method F is repeated. The -COOH group may be similarly converted to other ester groups.

The following representative drugs may be derivatized in this manner to the corresponding $[D\text{-}QC]^+$ and [D-DHC] compounds:

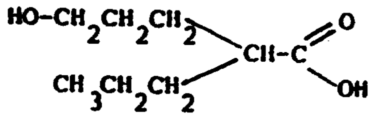

5-hydroxy-2-n-
propylpentanoic acid

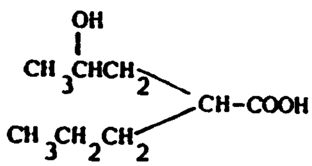

4-hydroxy-2-n-
propylpentanoic acid

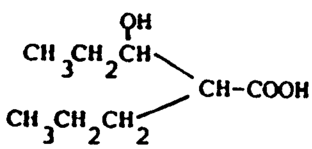

3-hydroxy-2-n-
propylpentanoic acid

III. Methods for Salt Formation

**Method H**

An ether solution of [D-DHC] is treated with an equivalent amount of anhydrous p-toluenesulfonic acid dissolved in dry ether. Mixing at room temperature is continued until the imminium salt precipitates out of solution. The salt is then removed by filtration.

Accordingly, provided hereby are not only a method and novel class of pro-prodrugs for the specific and/or target enhanced delivery to the brain of a wide variety of drug species via the bidirectional transport of the drug species into and out of the brain employing dihydropyridine $\rightleftharpoons$ pyridinium salt carrier redox systems, but also a system providing insight into the basic transport processes (both active and passive) of, and enzymatic activities in, the blood-brain barrier, as well as into the various processes

specific to the function of the brain. Again, another very significant aspect of the bioreversible redox delivery system according to this invention is the toxicity implication, for significantly reduced is systemic toxicity by accelerating the elimination of the drug/quaternary carrier system. And even central toxicity is reduced by providing for low level, sustained release of the active drug species in the brain. Low toxicity is provided both as regards the quaternary carrier and in combination with the drug.

**CLAIMS:**

1. A compound adapted for the site-specific/sustained delivery of a centrally acting drug species to the brain, said compound being a compound of the formula

[D-DHC] (I)

or a non-toxic pharmaceutically acceptable salt thereof, wherein [D] is a centrally acting drug species containing at least one reactive -OH, -COOH, -SH, -NH- or $-NH_2$ functional group (including compounds such as amides, imides, hydrazines and guanidines, as well as compounds having a primary or secondary amino group), and [DHC] is the reduced, biooxidizable, blood-brain barrier penetrating lipoidal form of a dihydropyridine $\rightleftharpoons$ pyridinium salt redox carrier comprising a dihydroquinoline or dihydroisoquinoline ring system, a carbon atom of the nitrogen-containing ring portion of said ring system being connected via a bridging group to a reactive -OH, -COOH, -SH, -NH- or $-NH_2$ functional group in the centrally acting drug species.

2. A compound according to Claim 1 wherein the centrally acting drug species [D] contains at least one reactive primary or secondary amino group and the redox carrier [DHC] together with the bridging group connecting the carrier to the amino group in [D] has the structural formula

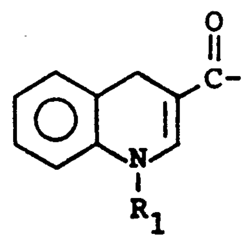

wherein $R_1$ is $C_1$-$C_7$ alkyl or $C_7$-$C_{10}$ aralkyl.

3. A compound according to Claim 2 wherein $R_1$ is methyl.

4. A compound according to Claim 1 wherein the centrally acting drug species [D] contains at least one reactive -OH group and the redox carrier [DHC] together with the bridging group connecting the carrier to the -OH group in [D] has the structural formula

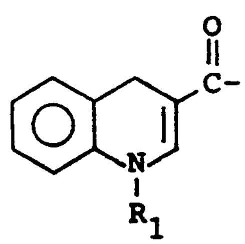

wherein $R_1$ is $C_1$-$C_7$ alkyl or $C_7$-$C_{10}$ aralkyl.

5. A compound according to Claim 4 wherein $R_1$ is methyl.

6. A compound according to Claim 1 wherein the centrally acting drug species [D] contains at least one reactive primary or secondary amino group and the redox carrier [DHC] together with the bridging group connecting the carrier to the amino group in [D] has the structural formula

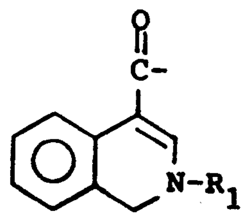

wherein $R_1$ is $C_1$-$C_7$ alkyl or $C_7$-$C_{10}$ aralkyl.

7. A compound according to Claim 6 wherein $R_1$ is methyl.

8. A compound according to Claim 1 wherein the centrally acting drug species [D] contains at least one reactive -OH group and the redox carrier [DHC] together with the bridging group connecting the carrier to the -OH group in [D] has the structural formula

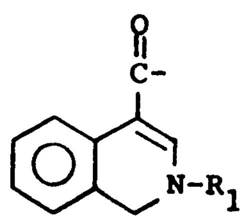

wherein $R_1$ is $C_1$-$C_7$ alkyl or $C_7$-$C_{10}$ aralkyl.

9. A compound according to Claim 8 wherein $R_1$ is methyl.

10. A compound according to Claim 1 wherein the centrally acting drug species [D] contains at least one reactive primary or secondary amino group and the redox carrier [DHC] together with the bridging group connecting the carrier to the amino group in [D] has the structural formula

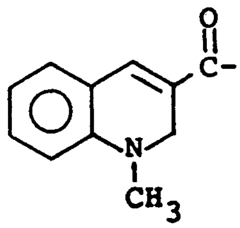

.

11. A compound according to Claim 1 wherein the centrally acting drug species [D] contains at least one reactive -OH group and the redox carrier [DHC] together with the bridging group connecting the carrier to the -OH group in [D] has the structural formula

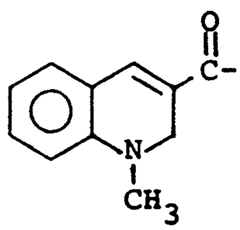

.

12. A compound according to Claim 1 wherein the centrally acting drug species [D] contains at least one reactive -SH group and the redox carrier [DHC] together with the bridging group connecting the carrier to the -SH group in [D] has the structural formula

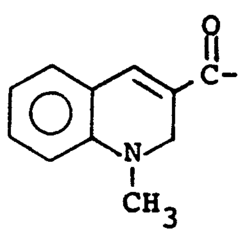

.

13. A compound according to Claim 1 wherein the centrally acting drug species [D] contains at least one reactive -NH- or $-NH_2$ functional group which is part of an amide or imide structure or which is a low pKa primary or secondary amino group, the redox carrier [DHC] comprises a dihydroquinoline ring system and the bridging group has the structure

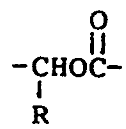

wherein R is $-CCl_3$, -H, 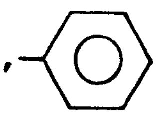 or $-CH_3$, the carbonyl function of the bridging group being connected to a carbon atom of the nitrogen-containing ring portion of the dihydroquinoline ring system.

14. A compound of the formula

$$[D\text{-}QC]^+ \qquad (II)$$

wherein [D] is a centrally acting drug species containing at least one reactive -OH, -COOH, -SH, -NH- or $-NH_2$ functional group (including compounds such as amides, imides, hydrazines and guanidines, as well as compounds having a primary or secondary amino group), and $[QC]^+$ is the hydrophilic, ionic, pyridinium salt form of a dihydropyridine ⇌ pyridinium salt redox carrier comprising a dihydroquinolinium or dihydroisoquinolinium ring system, a carbon atom of the nitrogen-containing ring portion of said ring system being connected via a bridging group to a reactive -OH, -COOH, -SH, -NH- or $-NH_2$ functional group in the centrally acting drug species.

15. A compound according to Claim 14 wherein the centrally acting drug species [D] contains at least one

reactive primary or secondary amino group and the redox carrier $[QC]^+$ together with the bridging group connecting the carrier to the amino group in [D] has the structural formula

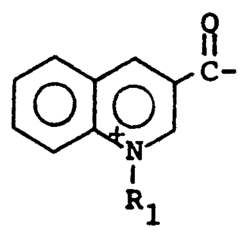

wherein $R_1$ is $C_1$-$C_7$ alkyl or $C_7$-$C_{10}$ aralkyl.

16. A compound according to Claim 15 wherein $R_1$ is methyl.

17. A compound according to Claim 14 wherein the centrally acting drug species [D] contains at least one reactive -OH group and the redox carrier $[QC]^+$ together with the bridging group connecting the carrier to the -OH group in [D] has the structural formula

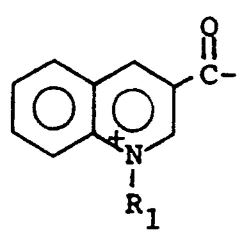

wherein $R_1$ is $C_1$-$C_7$ alkyl or $C_7$-$C_{10}$ aralkyl.

18. A compound according to Claim 17 wherein $R_1$ is methyl.

19. A compound according to Claim 14 wherein the centrally acting drug species [D] contains at least one reactive primary or secondary amino group and the redox carrier $[QC]^+$ together with the bridging group connecting the carrier to the amino group in [D] has the structural formula

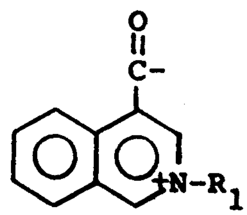

wherein $R_1$ is $C_1$-$C_7$ alkyl or $C_7$-$C_{10}$ aralkyl.

20. A compound according to Claim 19 wherein $R_1$ is methyl.

21. A compound according to Claim 14 wherein the centrally acting drug species [D] contains at least one reactive -OH group and the redox carrier $[QC]^+$ together with the bridging group connecting the carrier to the -OH group in [D] has the structural formula

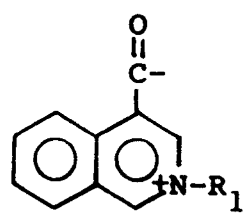

wherein $R_1$ is $C_1$-$C_7$ alkyl or $C_7$-$C_{10}$ aralkyl.

22. A compound according to Claim 21 wherein $R_1$ is methyl.

23. A compound according to Claim 14 wherein the centrally acting drug species [D] contains at least one reactive -NH- or $-NH_2$ functional group which is part of an amide or imide structure or which is a low pKa primary or secondary amino group, the redox carrier $[QC]^+$ comprises a dihydroquinolinium ring system and the bridging group has the structure

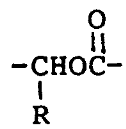

wherein R is $-CCl_3$, $-H$, 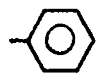 or $-CH_3$, the carbonyl function of the bridging group being connected to a carbon atom of the nitrogen-containing ring portion of the dihydroquinoline ring system.

24. A compound according to Claim 1 or 14 wherein [D] is a central neurotransmitter, an antiinflammatory steroid, a steroid sex hormone, an anticancer or antitumor agent, an antiviral agent, a tranquilizer, a memory enhancer or a hypotensive agent.

25. A compound according to Claim 1 or 14 wherein [D] is a sedative or hypnotic, an anticonvulsant or antiepileptic agent, a stimulant, a narcotic analgesic or antagonist, a dopaminergic agent or an antibiotic or antibacterial agent.

26. A compound according to Claim 1 or 14 wherein [D] is an amino acid or a small peptide containing 2 to 20 amino acid units.

27. A compound according to Claim 1 or 14, wherein [D] is a dopamine having the structural formula

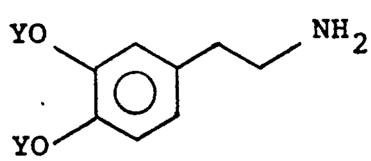

in which each Y is independently a hydrolytically or metabolically cleavable hydroxyl protective group.

28. A compound according to Claim 1 or 14, wherein [D] is GABA, glycine, glutamic acid, aspartic acid, dopamine, norephinephrine, epinephrine, serotonin, tryptamine, neurotensin, LHRH, somatostatin, an enkephalin, an endorphin, oxytocin M, vasopressin, hydrocortisone, betamethasone, dexamethasone, cortisone, flumethasone, fluprednisolone, meprednisone, methyl prednisolone, prednisolone, prednisone, triamcinolone, cortodoxone, fludrocortisone, fluandrenolone acetonide, paramethasone, testosterone, methyl testosterone, ethinyl estradiol, norgestrel, mestranol, norethindrone, ethisterone, estradiol, estriol, estrone, dimethisterone, allylestrenol, cingestol, ethynerone, lynestrenol, norgesterone, norvininsterone, ethynodiol, oxogestone, tigestol, quinestrol, Ara-AC, pentostatin, Ara-C, 3-deazaguanine, dihydro-5-azacytidine, tiazofurin, sangivamycin, Ara-A, 6-MMPR, PCNU, spiromustine, bisbenzimidazole, L-alanosine, DON, L-ICRF, trimethyl TMM, 5-methyl tetrahydrohomofolic acid, glyoxylic acid sulfonylhydrazone, DACH, SR-2555, SR-2508, desmethylmisonidazole, mitoxantrone, menogarol, aclacinomycin A, phyllanthoside, bactobolin, aphidocolin, homoharringtonine, levonantradol, acivicin, streptozotocin, hydroxyurea, chlorambucil, cyclophosphamide, uracil mustard, melphalan, 5-FUDR, cytosine arabinoside, 6-mercaptopurine, thioguanine, 5-azacytidine, methotrexate, doxorubicin, daunomycin, aminopterin, dactinomycin, mitomycin C, a podophyllotoxin derivative, ribavirin, acyclovir, amantadine, 5-amidino-2-(5-amidino-2-benzofuranyl)indole, 4',6-diimidazolino-2-phenylbenzo-(b)thiophene, 2-guanidino-4,5-di-n-propyloxazole, 2-guanidino-4,5-diphenyloxazole, 6[[(hydroxyimino)-phenyl]methyl]-1-[(1-methylethyl)sulfonyl]-1H-benzimidazol-2-amine, 5,7-dimethyl-2-β-D-ribofuranosyl-s-triazole(1,5-a)pyrimidine, 2-deoxy-D-glucose, glucosamine, 2-deoxy-2-fluoro-D-mannose, 6-amino-6-deoxy-D-glucose, phenyl-6-chloro-6-deoxy-β-D-glucopyranoside, (S)-9-(2,3-dihydroxypropyl)adenine, 6-azauridine, 5,6-dichloro-1-β-D-ribofuranosylbenzimidazole, diazepam, oxazepam, lorazepam, chlordiazepoxide, flurazepam, bromazepam, clorazepate, nitrazepam, temazepam, phenytoin, ethotoin, mephenytoin, acetophenazine, carphenazine, fluphenazine, perphenazine, piperacetazine, clonidine, methyldopa, bethanidine, debrisoquin, hydralazine, guanethidine, haloperidol, hydroxyzine, clopenthixol, γ-vinyl GABA, γ-acetylenic GABA, phenobarbital, amobarbital, butalbital, glutethimide, aminoglutethimide, methyprylon, valproic acid, 5-hydroxy-2-n-propylpentanoic acid, 4-hydroxy-2-n-propylpentanoic acid, 3-hydroxy-2-n-propylpentanoic acid, methamphetamine, phentermine, phenmetrazine, dextroamphetamine, levamphetamine, amphetamine, phenylethylamine, methylphenidate, tranylcypromine, protriptyline, opipramol, desipramine, nortriptyline, bemegride, amiphenazole, anileridine, hydromorphone, oxymorphone, apomorphine, levorphanol, morphine, pentazocine, codeine, oxycodone, naloxone, nalorphine, tryptophan, tryamine, levodopa, ampicillin, amoxicillin, oxacillin, carbenicillin, dicloxacillin, chlortetracycline, tetracycline, methacycline, nalidixic acid, cephalexin, cephalothin, cefoxitin, clindamycin, lincomycin, oxolinic acid, phenazopyridine, sulfadiazine, thiopental, benzestrol or diethylstilbestrol.